# EUROPEAN PATENT SPECIFICATION

(11) **EP 4 304 528 B1**
(45) Date of publication and mention of the grant of the patent: **25.03.2026**
(21) Application number: 22712771.9
(22) Date of filing: 10.03.2022
(51) Int. Cl.: A61F 2/24

(54) **ADJUSTABLE THREE-DIMENSIONAL ANNULOPLASTY RING**
EINSTELLBARER DREIDIMENSIONALER ANNULOPLASTIERING
ANNEAU D'ANNULOPLASTIE TRIDIMENSIONNEL RÉGLABLE

(30) Priority: 11.03.2021 US 202163159834 P
(43) Date of publication of application: 17.01.2024
(73) Proprietor: Edwards Lifesciences Corporation, Irvine, CA 92614-5688 (US)
(72) Inventor: CONKLIN, Brian, S., Orange, CA 92867 (US); MUNNELLY, Amy, E., Irvine, CA 92614 (US); DEHERRERA, Milton, Irvine, CA 92614 (US); CHEN, Harvey, H., Irvine, CA 92614 (US); LE, Jason, Thai, Irvine, CA 92614 (US); MIRAKI, Manouchehr, A., Irvine, CA 92614 (US); NGUYEN, Devin, D., Irvine, CA 92614 (US); JOSHI, Mrunalini, A., Irvine, CA 92614 (US); RODRIGUEZ, Rodolfo, Irvine, CA 92614 (US); KEAN, Ryan, Tran, Irvine, CA 92614 (US)
(74) Representative: Eisenführ Speiser
(86) International application number: PCT/US2022/019801
(87) International publication number: WO 2022/192572

(56) References cited:
- WO-A1-2014/144439
- WO-A1-99/04730

## Description

### TECHNICAL FIELD

The present disclosure relates generally to annuloplasty rings, and in particular to an adjustable mitral annuloplasty ring and delivery system.

### BACKGROUND

In vertebrate animals, the heart is a hollow muscular organ having four pumping chambers: the left and right atria and the left and right ventricles, each provided with its own one-way valve. The natural heart valves are identified as the aortic, mitral (or bicuspid), tricuspid and pulmonary, and each has flexible leaflets that coapt against each other to prevent reverse flow.

Various surgical techniques may be used to repair a diseased or damaged valve. A commonly used repair technique effective in treating incompetence is annuloplasty, which often involves reshaping or remodeling the annulus by attaching a prosthetic annuloplasty repair segment or ring thereto. The procedure is done with the heart stopped and the patient on cardiopulmonary bypass ("on pump"). For instance, the goal of a posterior mitral annulus repair is to bring the posterior mitral leaflet forward toward to the anterior leaflet to improve leaflet coaptation. Annuloplasty rings may be stiff, flexible or semi-rigid, and a "remodeling" annuloplasty ring typically has an inner core that is "generally rigid" or "semi-rigid" in that it will flex to a small extent but resist distortion when subjected to the stress imparted thereon by the mitral valve annulus of an operating human heart.

Currently, during a mitral valve repair procedure, the size of the annuloplasty ring is determined by comparing different sizer templates to the patient's anatomy until the surgeon determines which one looks correct based on, for example, anterior leaflet area or length, intercommissural distance, and so on. However, unlike for an aortic valve replacement, where the goal is to implant the largest valve that will safely fit the patient's anatomy, mitral repair procedures implant a repair device that is somewhat smaller than the annulus to reduce the perimeter, or, more importantly, the anterior-posterior (AP) diameter, of the valve and restore leaflet coaptation. The surgeon must make an "educated guess" as to how much reduction in size is appropriate for any given patient and their specific disease state. If the wrong size repair product is chosen, the result may be a poor outcome manifested by residual mitral regurgitation (MR), insufficient coaptation length, high pressure gradients, or systolic anterior motion (SAM). If any of these conditions are found once the patient is weaned off-pump, the surgeon must make the difficult decision of going back on pump, with its associated morbidity and mortality, or leaving the patient with a sub-optimal repair, and its associated sequalae.

Given the above challenges, it would be desirable to have an annuloplasty device that could be adjusted once the patient was weaned off-pump in order to fine-tune the AP diameter of the mitral valve in order to correct for small errors in the inherently imprecise sizing process. Such a ring would have the potential to reduce poor mitral valve repair outcomes and the need to go back on-pump in many cases. Once adjustments were made, the delivery system attachments could be disengaged, leaving the patient with a customized annuloplasty device that was tailored to their specific anatomy.

In attempts to vary the shape of the repair device, adjustable annuloplasty devices such as the CARDIOBAND^{®} mitral and tricuspid reconstruction systems are available from Edwards Lifesciences Corp. of Irvine, CA. Other adjustable annuloplasty rings may be seen in U.S. Patent Nos. 8,142,495, 8,349,002 and 9,107,749. Further, WO 2014/144439 A1 describes a device for treating a mitral valve defect. The device includes features that allow the device to conform to the actual pathology of the valve. The actual pathology of the valve is accommodated by providing for multiple dimensions of adjustability of the device, including adjustability of the size (e.g., diameter) of the device as well as adjustability of the elevation or inclination of one portion of the device (e.g., the portion supporting the posterior leaflet) with respect to another portion of the device.

Modern annuloplasty rings such as the Edwards Physio II^{®} annuloplasty ring have a very specific 3-dimensional shape, which has been shown to be important in maintaining and restoring anatomy as well as minimizing leaflet stresses. Adjustable devices have yet to successfully combine orifice downsizing with three-dimensional remodeling. Some are unduly complex while others have ring core elements that are completely rigid and do not appear to accommodate any shape change other than in the AP direction. Further, it is difficult to achieve optimal sizing of the mitral ring while the heart is on-pump; this sometimes leads to over- or under-sizing of the annulus, which may lead to post-operative complications.

Despite numerous designs presently available or proposed in the past, there is a need for an annuloplasty ring that may be shaped adjusted to effect repair of the malfunctioning valve while avoiding negative outcomes.

### SUMMARY

The present invention relates to an annuloplasty ring and shape adjustment system as defined by appended claim 1 and to a method of simulating implantation of an annuloplasty ring in a simulated patient at a native heart valve annulus and adjusting a peripheral shape of the annuloplasty ring as defined by appended claim 8.

The application discloses an adjustable annuloplasty ring system that is surgically implanted on-pump, but can be slightly adjusted off-pump on the beating heart in order to optimize the annular size and reduce complications due to under- or over-sizing of the ring. The adjustable annuloplasty ring and a method for adjusting and locking the device is disclosed. The ring is surgically implanted like a normal annuloplasty ring, and then the patient closed up, the heart restarted, and a size adjustment made under visualization, if needed.

Disclosed here is an adjustable 3D mitral annuloplasty ring core and delivery system. The disclosed system may use a simple ratcheting mechanism to change the A-P diameter of the ring in real-time during the procedure before or after the patient is weaned off-pump by applying simple displacements to a cable and housing arrangement via a delivery system. Once the surgeon is satisfied with the result, they can easily detach the delivery system from the implant and finish closing the patient. The disclosed system would be simpler and less expensive to manufacture than prior adjustable annuloplasty rings and have the added benefit of a true 3D annuloplasty ring shape.

In the invention as defined by claim 1, an annuloplasty ring and shape adjustment system comprises a three-dimensional annuloplasty ring defining a continuous peripheral shape around a central aperture. The annuloplasty ring has an inner core formed of an elastic-plastic material and a suture-permeable interface surrounding the inner core and extending around the peripheral shape. The inner core has an arcuate anterior segment that overlaps an arcuate posterior segment on opposite sides, the peripheral shape being a rounded D-shape in plan view wherein both the anterior segment and posterior segment rise up from the opposite sides to form a saddle shape. The anterior and posterior segments define a ratchet mechanism at overlapped regions on opposite sides such that a position of the anterior segment relative to the posterior segment may be changed and the peripheral shape of the annuloplasty ring may be adjusted. A pair of adjustment filaments adapted to pass through passages in one of the segments of the inner core are connected to and displace the other segment on opposite sides to adjust the peripheral shape of the annuloplasty ring. A plurality of anchoring sutures pass through the interface and secure the annuloplasty ring to a native heart valve annulus. A shape adjusting mechanism includes an access sheath having a length sufficient to extend from the native heart valve annulus through an access incision in the heart and out of the body. The pair of adjustment filaments pass through the access sheath. The shape adjusting mechanism further has a pair of tension adjusters and each of the adjustment filaments passing separately through an associated one of the tension adjusters to enable separate displacement of the adjustment filaments relative to the annuloplasty ring. The access sheath has sufficient column strength such that tension on either of the adjustment filaments caused by the associated tension adjuster adjusts the peripheral shape of the annuloplasty ring.

In the annuloplasty ring and shape adjustment system, the passages may be formed in the anterior segment and open to two closely-spaced entry holes on an atrial side of a midsection thereof. The passages are configured such that the adjustment filaments diverge from the entry holes and emerge on a radially outward side of the midsection, wherein secondary passages are formed in the anterior segment such that the adjustment filaments extend into inner channels formed by side sections of the anterior segment. Free ends of the posterior segment overlap the anterior segment in the inner channels, and the adjustment filaments attach to the free ends of the posterior segment.

The ratchet mechanism may include teeth on one segment engaging a detent on the other segment, and wherein each side section of the anterior segment includes the detent projecting into inner channels formed by side sections of the anterior segment. Further, the posterior segment includes a midsection and two side sections ending at the free ends, wherein each of the side sections of the posterior segment has a series of the teeth which the detents engage in each of the inner channels, the detents and teeth being arranged to prevent the side section of the posterior segment on each side from retracting from within the inner channels. The system may further include a biasing spring positioned within each of the inner channels that applies a compressive force to both the anterior segment and the posterior segment tending to force them apart.

The ratchet mechanism may include teeth on one segment engaging a detent on the other segment, or undulating bumps on one segment engaging mating undulating bumps on the other segment. The system may further include a compression sleeve surrounding each of the overlapped regions on opposite sides of the anterior and posterior segments.

An assembly of an adjustable mitral annuloplasty ring and delivery system is also disclosed herein (not part of the invention). The assembly comprises a three-dimensional annuloplasty ring defining a continuous peripheral shape around a central aperture. The annuloplasty ring has an inner core formed of an elastic-plastic material and a suture-permeable interface surrounding the inner core and extending around the peripheral shape. The inner core has an arcuate anterior segment that overlaps an arcuate posterior segment on opposite sides, the peripheral shape being a rounded D-shape in plan view wherein both the anterior segment and posterior segment rise up from the opposite sides to form a saddle shape. The anterior and posterior segments define a ratchet mechanism at overlapped regions on opposite sides such that a position of the anterior segment relative to the posterior segment may be changed and the peripheral shape of the annuloplasty ring may be adjusted. Finally, a pair of adjustment filaments are adapted to pass through a delivery system and through passages in one segment of the inner core, the filaments being connected to and displace the other segment on opposite sides to adjust the peripheral shape of the annuloplasty ring.

In the assembly above, the passages may be formed in the anterior segment and open to two closely-spaced entry holes on an atrial side of a midsection thereof, the passages being configured such that the adjustment filaments diverge from the entry holes and emerge on a radially outward side of the midsection. Secondary passages are also formed in the anterior segment such that the adjustment filaments extend into inner channels formed by side sections of the anterior segment, wherein free ends of the posterior segment overlap the anterior segment in the inner channels, and the adjustment filaments attach to the free ends of the posterior segment.

In the assembly of an adjustable mitral annuloplasty ring and delivery system, the ratchet mechanism may include teeth on one segment engaging a detent on the other segment, wherein each side section of the anterior segment includes the detent projecting into each of inner channels formed by side sections of the anterior segment. The posterior segment includes a midsection and two side sections ending at the free ends, wherein each of the side sections of the posterior segment has a series of the teeth which the detents engage in each of the inner channels, the detents and teeth being arranged to prevent the side section of the posterior segment on each side from retracting from within the inner channels. Additionally, a biasing spring positioned within each of the inner channels that applies a compressive force to both the anterior segment and the posterior segment tending to force them apart.

The ratchet mechanism may include teeth on one segment engaging a detent on the other segment, or undulating bumps on one segment engaging mating undulating bumps on the other segment. The system may further include a compression sleeve surrounding each of the overlapped regions on opposite sides of the anterior and posterior segments.

The delivery system further may include an access sheath through which the adjustment filaments pass proximally, the access sheath and adjustment filaments having sufficient lengths to extend out of the body. Each of the adjustment filaments pass separately through a tension adjuster to enable separate displacement of the adjustment filaments relative to the annuloplasty ring.

In either of the system or assembly described above, each of the tension adjusters preferably has a rotating dial to adjust the tension on the associated adjustment filaments, and a gauge calibrated to indicate distance that the associated adjustment filament is displaced. Furthermore, the system or assembly may include a hemostatic valve positioned at a proximal end of the access sheath, the delivery system also comprising a purse-string suture adapted to seal an access incision into the left atrium around the access sheath.

A method of implantation of an annuloplasty ring in a patient at a native heart valve annulus and adjusting a peripheral shape of the annuloplasty ring is also disclosed (not part of the invention). The method includes the steps of:
stopping a patient's heart and providing an access pathway to a native heart valve annulus though a body to the heart, including an access incision in the heart leading to a heart valve annulus;
looping a plurality of anchoring sutures through the heart valve annulus at spaced locations and retracting free ends of the anchoring sutures out of the body;
passing one end of each of the pairs of free ends of the anchoring sutures through a peripheral suture-permeable interface of an annuloplasty ring, the annuloplasty ring including an inner core defining a continuous peripheral shape around a central aperture and the interface closely surrounding the inner core and having a matching shape, the inner core having an arcuate anterior segment that overlaps an arcuate posterior segment on opposite sides, the peripheral shape being a rounded D-shape in plan view wherein both the anterior segment and posterior segment rise up from the opposite sides to form a saddle shape, and the anterior and posterior segments define a ratchet mechanism at overlapped regions on opposite sides such that a position of the anterior segment relative to the posterior segment may be changed and the peripheral shape of the annuloplasty ring may be adjusted, the annuloplasty ring further including a pair of adjustment filaments adapted to pass through passages in one segment of the inner core, the filaments being connected to and displace the other segment on opposite sides to adjust the peripheral shape of the annuloplasty ring;
passing both adjustment filaments through an access sheath, the access sheath and adjustment filaments having sufficient lengths to extend out of the body, each of the adjustment filaments passing separately through a tension adjuster to enable separate displacement of the adjustment filaments relative to the annuloplasty ring;
advancing the annuloplasty ring to the heart valve annulus using the access sheath along the anchoring sutures;
tying off the anchoring sutures on a proximal side of the annuloplasty ring;
sealing the access incision around the access sheath;
restarting the patient's heart;
externally visualizing blood flow through the heart valve annulus;
adjusting the tension on one or both of the adjustment filaments until regurgitation through the heart valve annulus is minimized;
severing the adjustment filaments on a proximal side of the annuloplasty ring; and
withdrawing the adjustment filaments and access sheath from the body and closing up the access pathway.

For the method, a hemostatic valve is desirably positioned at a proximal end of the access sheath, the system also comprising a purse-string suture adapted to seal an access incision into the left atrium around the access sheath. Each of the tension adjusters has a rotating dial to adjust the tension on the associated adjustment filaments, and a gauge calibrated to indicate distance that the associated adjustment filament is displaced. A knot pusher may be used to advance single knots between the adjustment filaments through the access sheath to create a compound knot between the adjustment filaments on a proximal side of the annuloplasty ring prior to severing the adjustment filaments.

All methods disclosed herein also encompass simulations of the methods, for example, for training; testing; demonstration; or device or procedure development. Methods for treating a patient can include simulating treatment on a simulated human or non-human patient, for example, an anthropomorphic ghost. Examples of suitable simulated patients can include both an entire body, any portion of a body, or at least a portion of an organ, for example, a heart. The simulations can be physical, virtual, or any combination thereof. Examples of physical simulations can include any combination of natural or manufactured whole human or animal cadavers, portions thereof, or cadaver organs. Virtual simulations can include any combination of virtual reality, projections onto a screen or on at least a portion of a physical simulation, or other *in silico* elements. Some simulations can include non-visual elements, for example, auditory, tactile, or olfactory stimuli.

Another annuloplasty ring has a main core segment and at least one adjustable segment in the P2 region of the mitral annulus. The main core segment overlaps with the adjustable core segment. The overlapping ends are covered loosely with shrink tubing to hold the main core segment and adjustable core segment in place, while also allowing them to move relative to each other when a force is applied. Alternatively, the adjustment segment can be a sleeve that fits over the ends of the main core segment. Each end of the main core segment has a filament attached; the filament can be pulled in tension to adjust the size/shape of the ring.

After implantation, a small port is made in the left atrium, and the adjustment filaments are threaded through the port via a catheter. The heart is revived, and flow through the mitral valve can be viewed via echo. The adjustment filaments can then be pulled to customize the size and shape of the ring in real time while viewing the regurgitation on echo. When the optimal result is achieved, each adjustment filament is locked via an automatic fastener device (such as Sutrafix) which is placed through the atrial port and actuated to lock and cut the adjustment filaments. The atrial port is removed and the atrium closed to complete the procedure.

Instead of just one adjustable segment, the ring may be designed to have multiple segments that can slide past each other to provide finer adjustment in specific areas of the ring. The ring may be attached to a holder system which is manipulated at the beginning of the procedure to put pretension on the ring adjustment filaments. The ring is implanted with pretension so that during sizing, the ring can be made larger by releasing the pretension or smaller by applying more tension to the filaments. The ring holder system may have a knob that can be turned to tighten or loosen filaments by a specific amount, to achieve more precise control of sizing.

A further understanding of the nature and advantages will become apparent by reference to the remaining portions of the specification and drawings.

### BRIEF DESCRIPTION OF THE DRAWINGS

Features and advantages of the present disclosure will become appreciated as the same become better understood with reference to the specification, claims, and appended drawings wherein:
Figure 1 is an atrial plan view of a mitral valve and leaflets indicating common nomenclature initials for regular anatomical features;
Figure 2 is a schematic view of the three-dimensional shape of the mitral annulus with several anatomical landmarks indicated;
Figures 3A-3E are orthogonal and sectional views of the exemplary annuloplasty ring of the present application;
Figure 4 is a plan view of an adjustable inner ring core of the exemplary annuloplasty ring shown attached to an adjustment mechanism incorporated into a delivery system;
Figure 5 is a perspective assembled view of the inner ring core, and Figure 6 is an exploded view thereof;
Figures 7-9 are plan views of a first inner ring core after having been size adjusted superimposed over a second inner ring core that remains in a neutral shape, and Figures 7A, 8A, 9A and 9B are enlargements of side portions thereof;
Figure 10 is a plan view of an alternative adjustable inner ring core for the exemplary annuloplasty ring, and Figure 11 is an exploded view thereof;
Figures 12-13 are plan views of a first alternative adjustable inner ring core after having been size adjusted superimposed over a second one that remains in a neutral shape, and Figures 12A and 13A are enlargements of side portions thereof;
Figure 14 is a schematic view of a ring size adjustment system for the three-dimensional annuloplasty ring;
Figures 15A and 15B show several steps for securing the annuloplasty ring after a ring size adjustment, and Figure 16 is an enlarged view of a knot pusher used in the process;
Figures 17A and 17B are plan views of a further alternative inner ring core shown in initial and constricted states, respectively, and a distal end of a delivery system therefor;
Figure 18 is an exploded view of the alternative inner ring core and delivery system of Figure 17A;
Figures 19A and 19B are plan views of another alternative annuloplasty ring shown in initial and constricted states, respectively, and a delivery system therefor;
Figure 20 is a perspective assembled view of still another adjustable annuloplasty ring disclosed herein, and Figure 21 is an exploded view thereof;
Figure 22A is a plan view of one segment of the inner ring core of the adjustable annuloplasty ring of Figure 20, shown in both relaxed and neutral states, and Figure 22B schematically shows various constricted shapes that the segment may attain;
Figure 23 is a plan view of one segment of an adjustable inner ring core with adjustment filaments attached to free ends, and Figure 23A is a perspective view of one free end of the segment and one way to attach a filament;
Figure 24 is a perspective view of a free end of the segment in Figure 23 showing an alternative attachment configuration;
Figure 25 is a perspective view of another adjustable inner ring core of the present application, and Figure 25A is an enlargement thereof showing a free end of a segment of the inner ring core with an adjustment filament;
Figure 26A is an enlarged perspective view similar to Figure 25A showing a different core segment, and Figure 26B shows one way to couple the segment to the adjustment filament.

### DETAILED DESCRIPTION

The right ventricle RV and left ventricle LV are separated from the right atrium RA and left atrium LA, respectively, by the tricuspid valve TV and mitral valve MV; *e.g.,* the atrioventricular valves. Though correction of the mitral annulus is the primary focus of the present application, it should be understood that certain characteristics of the annuloplasty rings described herein may equally be used to treat the tricuspid valve TV, and thus the claims should not be constrained to the mitral ring unless expressly limited.

The term "axis" in reference to the illustrated annuloplasty rings, and other non-circular or non-planar rings, refers to a line generally through the centroid of the ring periphery when viewed in plan view. "Axial" or the direction of the "axis" can also be viewed as being parallel to the average direction of blood flow within the valve orifice and thus within the ring when implanted therein. Stated another way, an implanted mitral ring orients about a central flow axis aligned along an average direction of blood flow through the mitral annulus from the left atrium to the left ventricle. The plan views of the annuloplasty rings illustrated herein are as looking from the atrial side in the direction of blood flow. For the purpose of orientation, therefore, the atrial side of the ring is up in the ventricular site is down.

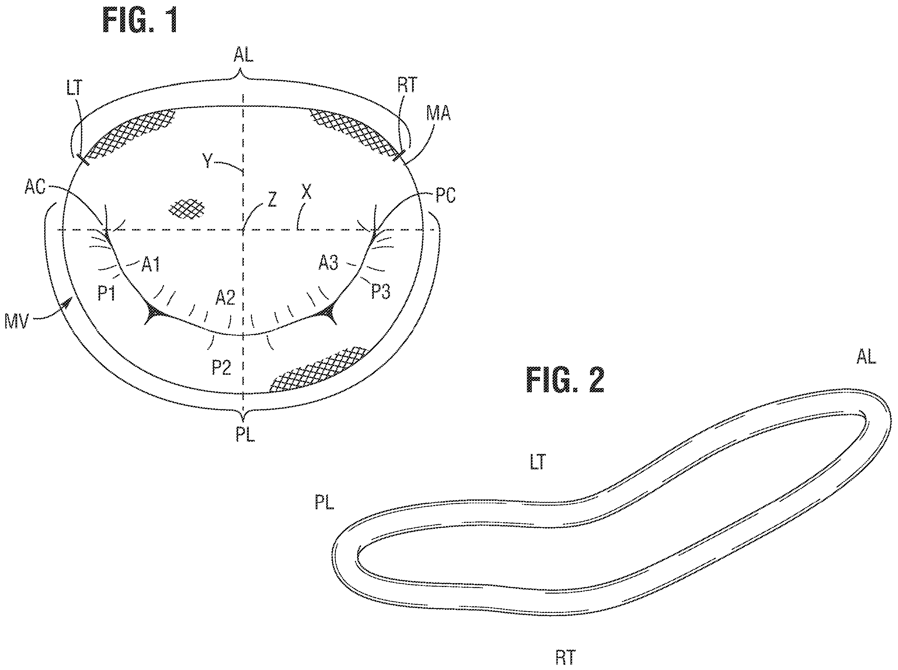

Figure 1 is a schematic perspective view from the atrial side of a mitral valve MV with posterior being down and anterior being up. The mitral valve MV primarily comprises a pair of coapting leaflets - an anterior leaflet AL and a posterior leaflet PL - secured around their outer edges to a fibrous mitral annulus MA. The surrounding mitral annulus MA is often described as D-shaped with a somewhat straighter side adjacent the anterior leaflet AL and a more rounded or convex side adjacent the posterior leaflet PL. The mitral annulus MA is typically viewed as having a major axis **X** that intersects both the first and third posterior scallops P1 and P3, approximately at the commissures AC, PC, and a minor axis **Y** that intersects and generally bisects the middle posterior scallop P2. A central flow axis **Z** is arbitrarily defined at the intersection of the major and minor axes **X, Y.**

The leaflets are shaped such that the line of coaptation resembles a smile that approximately parallels the posterior aspect of the mitral annulus MA. The anterior leaflet AL spans a smaller peripheral aspect around the mitral annulus MA than the posterior leaflet PL, but the anterior leaflet AL has a convex free edge that extends farther into the orifice defined by the mitral annulus MA. The posterior leaflet PL, on the other hand, has a generally concave free edge. Two commissures - an anterior commissure AC and a posterior commissure PC - generally defined the intersection of the line of coaptation between the two leaflets AL, PL and the mitral annulus MA. The posterior leaflet is divided into three scallops or cusps, sometimes identified as P1, P2, and P3, starting from the anterior commissure AC and continuing in a counterclockwise direction to the posterior commissure PC. Per convention, a major axis of the mitral annulus intersects both the first and third posterior scallops P1 and P3, approximately at the commissures AC, PC, and a minor axis intersects and generally bisects the middle posterior scallop P2. The anterior leaflet also features scallops or regions labeled A1, A2, and A3 as indicated in Figure 1.

As illustrated, the mitral annulus has a kidney or rounded D-shape around its periphery. The mitral anterior leaflet AL attaches to a somewhat straight anterior fibrous portion of the mitral annulus, which makes up about one-third of the total mitral annulus circumference. The anterior fibrous annulus, the two ends of which are called the fibrous left and right trigones LT, RT, forms part of the central fibrous skeleton of the heart. The arcuate muscular portion of the mitral annulus constitutes the remainder of the mitral annulus, and the posterior leaflet PL attaches thereto. The anterior commissure AC and the posterior commissure PC are located just posterior to each fibrous trigone.

Figure 2 is a schematic view of the three-dimensional shape of the mitral annulus with several anatomical landmarks indicated. In particular, the anterior leaflet AL is separated from the posterior leaflet PL by the left and right trigones LT, RT. The three-dimensional shape is somewhat like a saddle, with the trigones LT, RT in a valley and the leaflets AL, PL rising up.

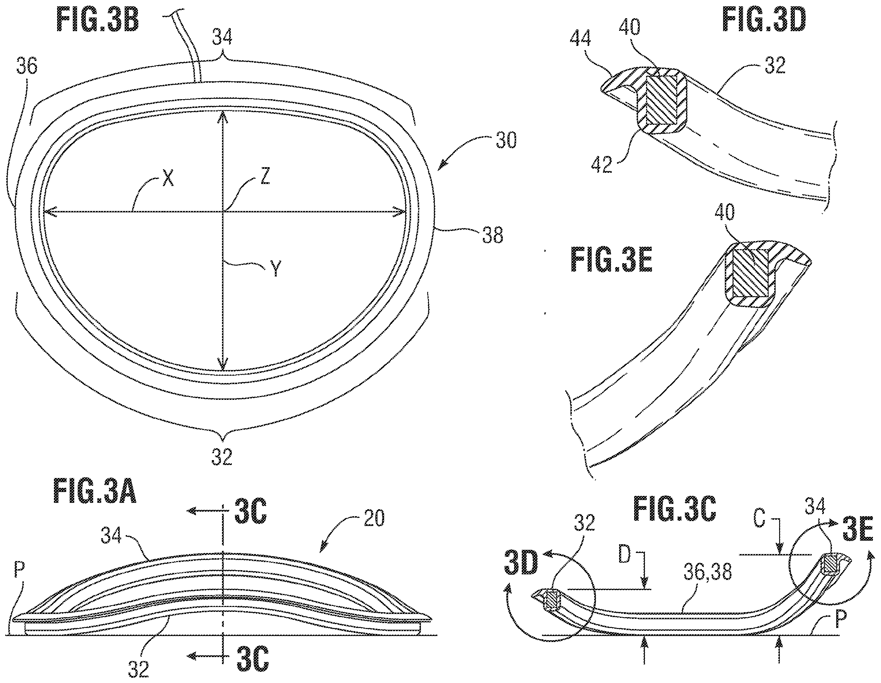

As seen in Figures 3A-3C, the exemplary annuloplasty ring 30 has a posterior portion 32 opposite an anterior portion 34, with side segments 36, 38 in between. Some nomenclature has the posterior portion 32 extending roughly around the posterior leaflet PL (Figure 2), extending between the leaflet commissures AC, PC, but generally the posterior portion 32 is bisected by the minor axis Y and extends at least around the middle posterior scallop P2 of the posterior leaflet PL. A major axis **X** and a minor axis **Y** are indicated which, when the annuloplasty ring 30 is implanted, coincide with the same axes of the native mitral annulus, such that blood will flow into the page generally parallel to a flow axis **Z** through the middle of the ring. The plan view shape of the annuloplasty ring 30 is kidney or rounded D-shaped so as to conform to the peripheral shape of the typical mitral annulus.

The annuloplasty ring 30 may be three-dimensional with an upward bow in the posterior portion 32 as well as an upward bow in the anterior portion 34, as seen in Figures 3A and 3C. Preferably the anterior portion 34 bows upward a distance **C** from a reference plane **P** more than an upward bow **D** of the anterior portion 34. The side segments 36, 38 may lie in the reference plane **P** such that the ring 30 is partially planar with the two opposite upward bows, to form somewhat of a saddle shape so as to better conform to the native mitral annulus, as depicted in Figure 2. The shape of the ring 30 is similar to that of the Physio II^{®} annuloplasty ring available from Edwards Lifesciences of Irvine, CA.

Figures 3D and 3E are enlarged sectional views of the annuloplasty ring 30 as seen in section in Figure 3C. In a preferred example, the ring construction includes an adjustable inner core 40 formed by relatively rigid segments, as will be described, and surrounded by a suture-permeable interface. In the illustrated example, the inner core 40 at least along the minor axis is solid and rectangular, with a greater axial dimension than radial dimension. As will be explained below, the material of the inner core 40 is an elastic-plastic material such as stainless steel or titanium alloy which permanently deforms after reaching a yield stress. Rather than an elastic plastic metal, an elastic-plastic polymer such as nylon (polyamide), polyacetal (e.g., DELRIN^{®} polyoxymethylene), or the like may also be utilized. The annuloplasty ring 30 is thus a remodeling ring.

The suture-permeable interface may include an elastomeric sleeve 42 closely surrounding the core and a fabric outer cover (not shown), for example, a polyethylene terephthalate (PET) fabric cover. In the preferred example the elastomeric sleeve 42, which may be silicone rubber, is generally tubular and molded to have a radially outwardly-extending flange 44 to facilitate suturing of the ring 30 to the mitral annulus. The ring 30 may be secured with sutures, staples, or other such devices to an inside fibrous ledge of the mitral annulus. In a typical procedure, the surgeon anchors an array of sutures through the annulus and then threads them through corresponding locations around the interface on the outside of the ring 30. The ring is parachuted down the suture array to be seated at the annulus before tying off the sutures.

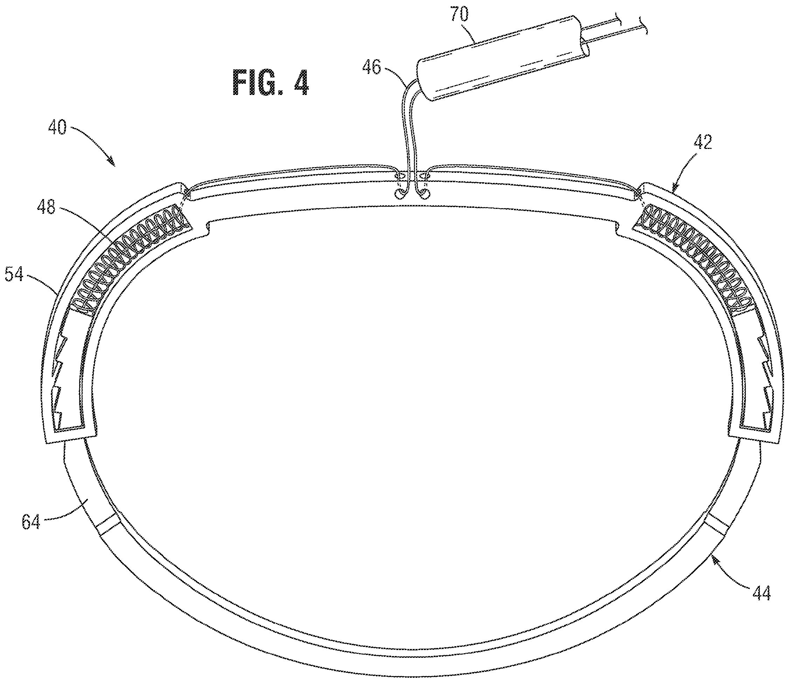

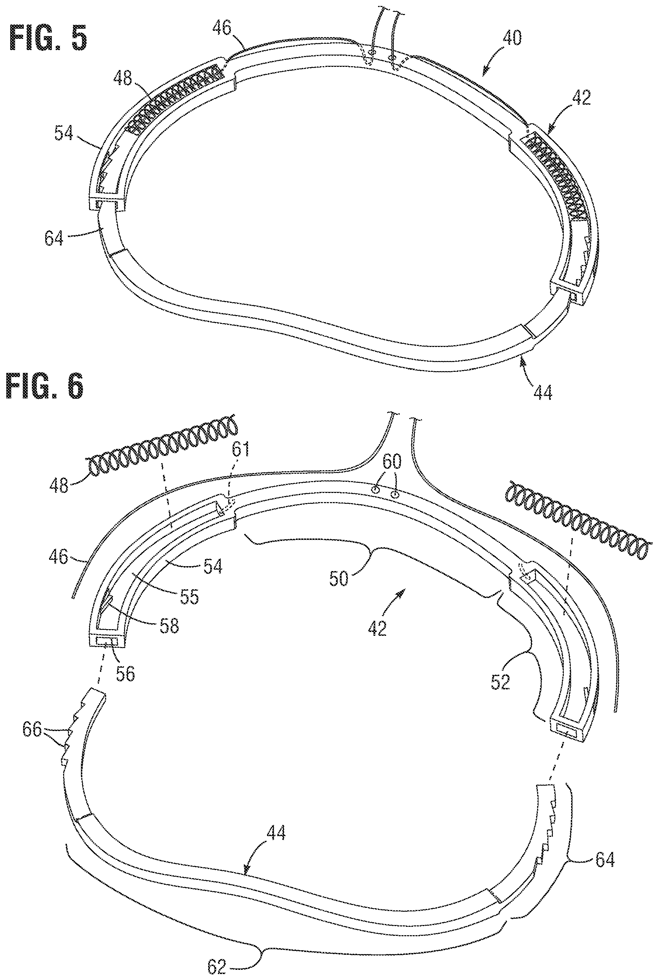

As seen in the plan view of Figure 4, the annuloplasty ring core 40 is formed primarily by two overlapping pieces: an anterior segment 42 and a posterior segment 44. Figure 5 is a perspective view of the inner ring core 40, and Figure 6 is an exploded view thereof. The other pieces of the ring core 40 include a pair of adjustment filaments 46 that are routed through the anterior segment 42 and attached to the posterior segment 44, and a pair of biasing springs 48 that are held within the anterior segment 42 and act on the posterior segment 44.

With reference to the exploded view of Figure 6, the anterior segment 42 comprises a midsection 50 and two side sections 52 that extend outward therefrom. Each side segment 52 has a pair of parallel side walls 54 that define an inner channel 55 therebetween, each inner channel terminating at an open end 56 at the free or terminal end of the respective side section. As will be explained, free ends of the posterior segment 44 are received in the open ends 56 so as to slide within the inner channels 55. A ratchet pawl or detent 58 extends inward from one of the side walls 54 into the inner channel 55, and could be formed simply by bending a segment of the wall inward. The detents 58 are angled so as to prevent the posterior segment 44 from expanding relative to the anterior segment 42, as will be explained below.

The midsection 50 also has a pair of closely-spaced entry holes 60 on an atrial side which lead to internal passages (not shown) that open to the radially outer face of the midsection. The adjustment filaments 46 pass down into the entry holes 60 and diverge through the internal passages to emerge and extend circumferentially around the midsection 50 until they pass through secondary apertures 61 that lead to the inner channels 55. Each of the adjustment filaments 46 attaches to a free end of the posterior segment 44 within the inner channels 55. The filaments 46 may be tied to a feature at the free ends of the posterior segment 44, such as through holes or eyelets, or maybe secured via heat bonding, adhesive, or the like.

The posterior segment 44 also includes a midsection 62 and two side sections 64 that extends outward therefrom. Each side section 64 has serrations or a series of evenly-spaced teeth 66 defined on an outer face and extending to the free ends. The side sections 64 are sized to extend into the open ends 56 of the inner channels 55. The teeth 66 eventually engage the detent 58 which prevents retraction of the side sections 64 from the inner channels 55. More particularly, the detent 58 is angled toward the midsection 50 of the anterior segment 42 and flexes outward upon passage of the side sections 64, and the teeth 66 are angled so that the detente 58 springs back inward into the indents formed between the teeth.

The biasing springs 48 reside within the inner channels 55 and apply a compressive force to both the closed end of the channel and a free end of the posterior segment 44, as shown in Figure 5. The springs 48 thus maintain a slight load between the anterior and posterior segments 42, 44 tending to force them apart. The detents 58 prevent the posterior segment 44 from expanding out of the inner channels 55, and the springs 48 resist opposite motion and help prevent inadvertent shape changes during handling and implantation. Additionally, the springs 48 facilitate expansion of the diameter when the ratchet detents 58 are held in the open position.

The ring core 40 is a continuous, closed rounded D-shape, and thus the overlapping segments 42, 44 are entirely arcuate and convex outward, even though some portions have greater curvatures than others. Namely, as seen best in plan view, the midsection 50 of the anterior segment 42 is substantially straighter than the two side sections 52. Likewise, the midsection 62 of the posterior segment 44 has a greater radius of curvature than the adjacent side sections 64.

Figures 4 and 5 show a neutral position for the ring core 40 with the detents 58 positioned in a second indent from the free end of the side sections 64 of the anterior segment 44. This "neutral" position is calibrated so that the ring core 40 has a size that matches the desired size for an annuloplasty ring intended to be implanted at a particularly sized mitral annulus. That is, prior to any annuloplasty surgery, the surgeon will measure the size of the native mitral annulus, typically by measuring directly across the major axis, and then select a desired size of ring. Annulus sizes are often measured in 2 mm increments, for example, between 26-40 mm, such that annuloplasty rings are supplied and labeled in sizes 26-28-30-32-34-36-38-40. The "neutral" sizes of the ring cores 40 are calibrated to produce these labeled ring sizes. Due to their adjustability, however, each ring core 40 can be adjusted to alter the size of one or both sides, as will be shown. In this way, the surgeon could either enlarge the ring core and reduce coaptation if there was an issue with systolic anterior motion (SAM), for example, or reduce the circumference if there was residual mitral regurgitation (MR). The spacing of the teeth 66 could be about 1 mm, for example, allowing for relatively high resolution size adjustments of the ring core.

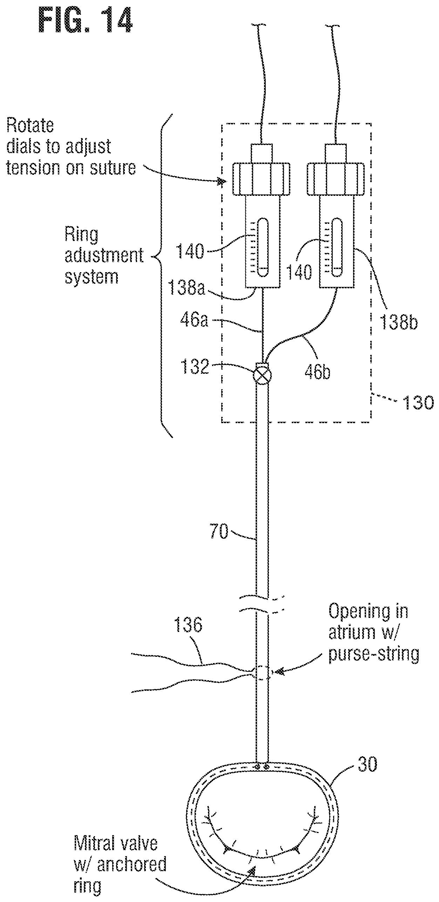

The adjustment filaments 46 may be formed by cables or sutures and attach to the free ends of the posterior segment 42, as shown in Figures 4 and 5. As mentioned, the filaments 46 are routed through the anterior segment 42 and then to a delivery system. Figure 4 schematically illustrates a distal end of an access sheath 70 which forms a part of the delivery system. As shown schematically in Figure 14, the filaments 46 extend proximally through the access sheath 70 to a ring size adjustment system 130 which may be used to adjust the size of the annuloplasty ring 30 post-implant, as explained below.

Pulling on the adjustment filaments 46 pulls on the free ends of the posterior segment 42, which causes them to be pulled further into the inner channels 55 of the anterior segment 42. Because of the one-way nature of the engagement between the detent 58 and the teeth 66, the free ends of the posterior segment 42 thus extend incrementally further into the inner channels 55, thus reducing the circumference (or AP diameter) of the ring core 40. As mentioned, one or both side sections 64 of the posterior segment may be displaced.

The midsection 50 of the anterior segment 42 is more flexible than the side walls 54. This is necessary as there is a slight change to the overall D-shape of the ring when the AP diameter changes by sliding the ends of the posterior segment 44 further into the anterior segment 42. Likewise, the entire posterior segment 42 is somewhat flexible for the same reason. The cross-sectional dimensions and material of the inner core 40 (an elastic-plastic metal such as stainless steel or titanium alloy) is such that some flexing of the segments 42, 44 occurs during size adjustments, though the remodeling capacity of the inner core 40 is maintained.

Although not shown, an additional cable or suture could be routed on each side that would hold the ratchet detents 58 in an open position until it was desired to lock the diameter. In this way, adjustments could reversibly be made both increasing and decreasing the AP diameter until the surgeon was satisfied with the result. The ring could then be locked at that diameter by releasing the ratchet detent cables. This could be done on a beating heart using TEE to monitor the hemodynamic result.

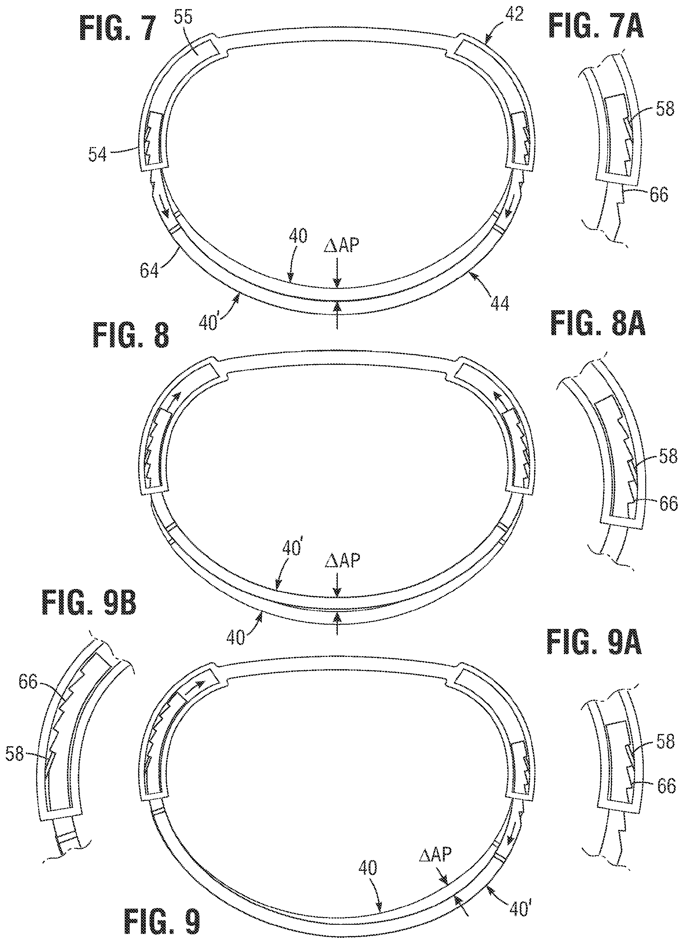

Figures 7-9 are plan views of a first inner ring core 40' after having been size adjusted superimposed onto a second inner ring core 40 that remains in a neutral shape, and Figures 7A, 8A, 9A and 9B are enlargements of side portions thereof. Specifically, Figure 7 shows a first inner ring core 40' expanded in the AP direction by one tooth 66 per side on top of a second ring core 40 that remains in the neutral configuration. That is, the detent 58 is shown in Figure 7A having been displaced from the second indent to the first indent between teeth 66 and closest to the free ends of the side segments 64. Again, this may be accomplished by providing a mechanism for flexing the detente 58 outwardly, such as a secondary cable (not shown). The increase in the anterior-posterior dimension (AP diameter) is indicated as ΔAP, and may be between about 1-2 millimeters.

Figure 8 shows the lower ring core 40 in the neutral configuration with the top ring core 40' in a partially collapsed configuration. More particularly, as seen in Figure 8A, the detent 58 has moved from the neutral position in the second indent to the third indent away from the free end of the posterior segment 44. Given the one-way nature of the detent 58, this ring reduction is accomplished by pulling on the adjustment filament 46 on that side which moves the side section 64 farther into the internal channel 55 against the bias of the spring 48. The decrease in the anterior-posterior dimension (AP diameter) is indicated as ΔAP, and may be between about 1-2 millimeters.

Figure 9 shows another potential advantage of the adjustability of the ring core 40; the ability to asymmetrically change shape. In this case, the right side is fully expanded while the left side is fully contracted, as indicated by the enlargements of Figures 9A and 9B. This results in an asymmetric shape as shown. This could be useful in the case of an eccentric jet on, for example, the A3/P3 side of the valve. As before, the changes in the anterior-posterior dimension (AP diameter) on the right side is indicated as ΔAP.

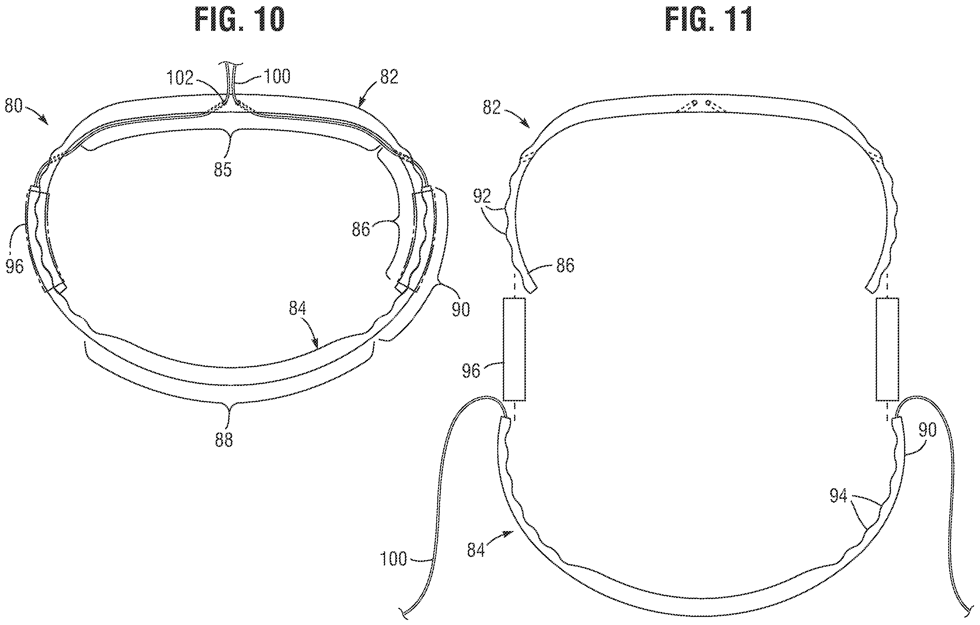

Figure 10 is a plan view of an alternative adjustable inner ring core 80 for the exemplary annuloplasty ring 30, and Figure 11 is an exploded view thereof. Again, the ring core 80 has an anterior segment 82 overlapping a posterior segment 84. The anterior segment 82 includes a midsection 85 and a pair of side sections 86 that extend outward therefrom. The posterior segment 84 has a midsection 88 and a pair of side sections 90. The side sections 86, 90 of the two segments overlap with the posterior segment 84 outside of the anterior segment 82. A series of undulating bumps 92 are provided on the outer face of the side sections 86 which mate or interlock with a series of undulating bumps 94 on the interface of the side sections 90. Of course, anterior segment 82 could overlap to the outside of the posterior segment 84 such that the undulating bumps 92, 94 were on opposite faces.

The overlapping side regions of the two segments 82, 84 are surrounded by a compression sleeve 96, such as could be formed by a length of shrink tubing. The compression sleeve 96 applies a certain amount of force holding the interlocking bumps 92, 94 relatively fixed, and requiring a predetermined threshold shear force to move them apart.

Figure 10 shows the compression sleeve 96 in place and a pair of adjustment filaments 100 routed through the anterior segment 82. Specifically, the filaments 100 pass in through a hole 102 in the atrial face of the anterior segment 82 and emerge at an inner face of the ring core 80. The adjustment filaments 100 travel around the circumference of the anterior segment 82 it passes outward through passages in the ring core to be connected to the free ends of the posterior segment 84. Consequently, applying sufficient tension to the adjustment filaments 100 would overcome the threshold shear force generated by the compression sleeves 96 and allow the bumps 92, 94 to slide to the next in the series. As such, the ring core 80 can be incrementally reduced in size, such as in 1 mm steps.

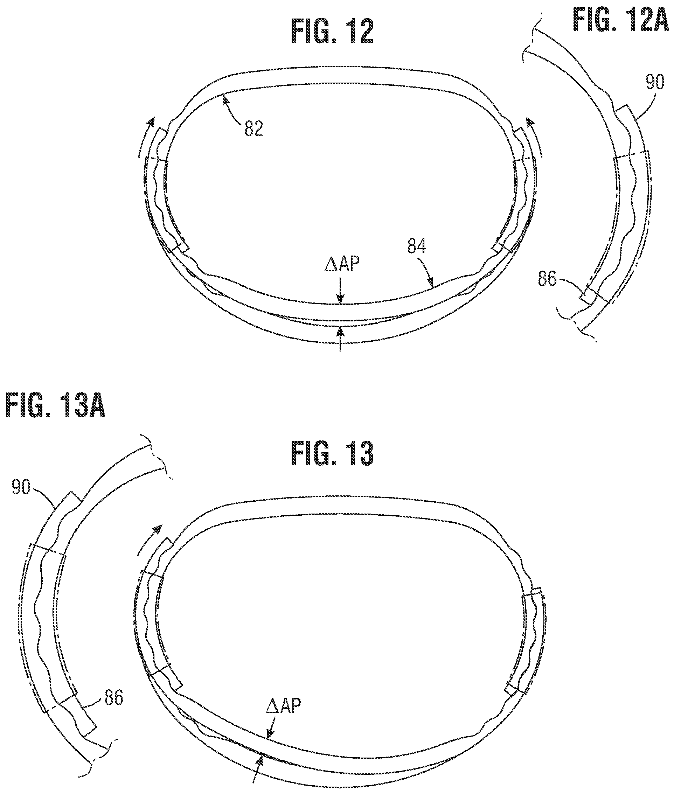

Figures 12-13 are plan views of a first alternative adjustable inner ring core 80' after having been size adjusted superimposed onto a second one 80 that remains in a neutral shape, and Figures 12A and 13A are enlargements are enlargements of side portions thereof. Specifically, Figure 12 shows a first inner ring core 80' in a partially collapsed configuration. More particularly, as seen in Figure 12A, the bumps 94 on the side sections 90 of the posterior segment 84 have moved upward to the next bump 92 on the anterior segment 82. This results in a reduction in the A-P dimension in the middle of the ring core 80' of between about 1-2 mm, or potentially more.

Figure 13 shows another potential advantage of the adjustability of the ring core 80; the ability to asymmetrically change shape. In this case, the right side remains neutral while the left side is contracted, as indicated by the enlargement of Figure 13A. This results in an asymmetric shape as shown with a reduction in the A-P dimension on the left side of between 1-2 mm.

It should be noted that, although all the CAD models and prototypes shown are 2D in shape, the same principals could also apply to fully 3D rings with a physiologic shape. Other variations of ratchet and detent mechanisms should also be considered as included in this disclosure. This procedure could be done initially while the patient was on-pump using the saline test to evaluate the repair, and then verified and adjusted further off-pump, or the entire adjustment procedure could be done on- or off-pump.

Figure 14 is a schematic view of the ring size adjustment system 130 for the adjustment filaments 46a, 46b. The access sheath 70 is shown advanced into contact with the atrial face of the anterior portion of the annuloplasty ring 30. It should be noted that although the ring size adjustment system 130 is shown in conjunction with the first example of the annuloplasty ring 30, the same system may be used to adjust the size of the second example of the annuloplasty ring 80. Indeed, the ring size adjustment system 130 may be used with any of the delivery systems and adjustable rings disclosed herein.

The access sheath 70 is outfitted with a hemostatic valve (schematically indicated at 132) to prevent blood from freely flowing through it when off-pump adjustments are being made. The access sheath 70 extends from outside of the body into proximity with the mitral valve, such as into the left atrium. One access route is through a small hole formed in the left atrial wall, and a hemostatic seal formed using a purse-string suture 136 as shown. With the purse-string suture 136 tightened around the access sheath 70, the heart may be restarted without risk of leakage around the sheath.

The aforementioned adjustment filaments 46a, 46b diverge at the proximal end of the access sheath 70 and pass through separate tension adjusters 138a, 138b. The dashed outline indicates a housing of some sort which maintains rigidity between each of the tension adjusters 138a, 138b and the access sheath 70 to allow the tension adjusters to increase or decrease tension on each of the adjustment filaments 46a, 46b relative to the annuloplasty ring 30. Contact of the access sheath 70 against the ring 30 provides a brace to enable adjustment of each of the adjustment filaments 46a, 46b. That is, the access sheath 70 will resist the tension applied to the adjustment filaments 46a, 46b by carrying a compressive load, but will be flexible to allow for positioning and movement.

In the illustrated example, the tension adjusters 138a, 138b have rotating dials which gradually adjust the tension on the corresponding suture, and a gauge 140 calibrated to indicate distance. This provides an indication of how much each tension adjuster 138a, 138b is pulled which, in turn, indicates how much of a size adjustment on each side of the ring is made. Of course, sliders or other tension adjusters can be utilized. While the patient's heart is beating and the mitral valve is subjected to external visualization, such as fluoroscopy, tension on each of the adjustment filaments 46a, 46b may be adjusted in coordination until regurgitation through the mitral valve is minimized.

In a conventional preliminary step, the ring 30 may be secured with sutures, staples, or other such devices to an inside fibrous ledge of the mitral annulus. Typically, the surgeon anchors an array of sutures through the annulus and then threads them through corresponding locations around the interface on the outside of the ring 30. The ring is parachuted down the suture array to be seated at the annulus before tying off the sutures. Figure 14 is a snapshot after the annuloplasty ring 30 has been parachuted down the array of anchoring sutures into contact with the mitral annulus. Each of the anchoring sutures is then tied off above the ring 30 at knots (not shown) spaced around the periphery of the ring.

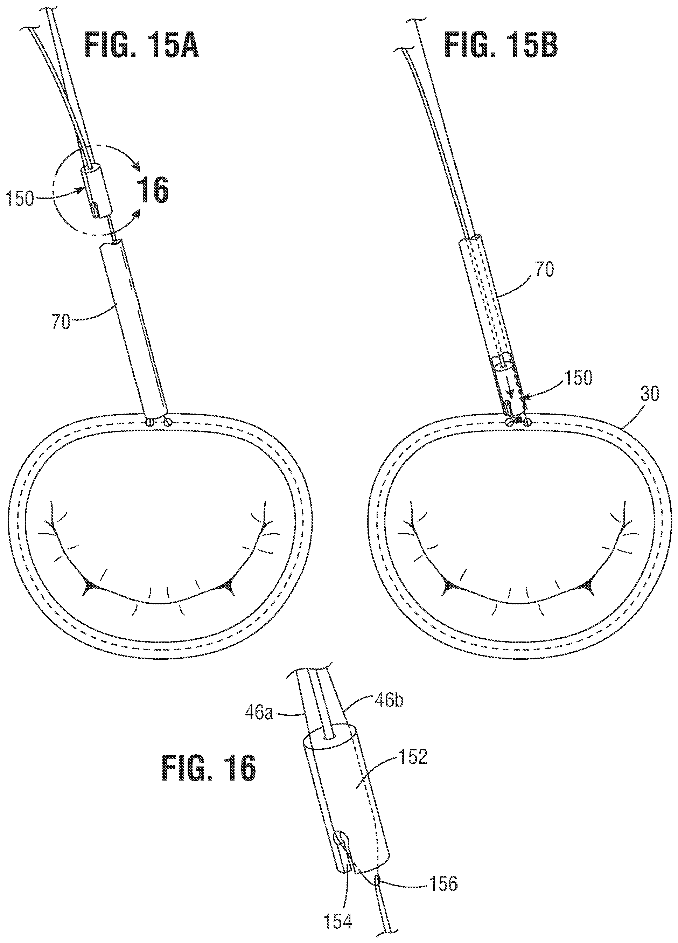

Figures 15A and 15B show several steps for securing the annuloplasty ring 30 after a ring size adjustment. Tension is pulled on the adjustment filaments 46a, 46b via the tension adjusters 138a, 138b while blood flow through the mitral valve MV is visualized until no or an acceptable level of regurgitation is seen. At this point, loosely-formed single knots are sequentially formed between the adjustment filaments 46a, 46b and advanced to the annuloplasty ring 30. This operation is accomplished outside the body for convenience.

Figure 15A shows engagement of a knot pusher 150 with a knot between the adjustment filaments 46a, 46b. The knot pusher 150 is shown in Figure 16 and has an enlarged head 152 with a distal end bifurcated by a lateral slit 154 in which the loose single knot 156 may be captured with free lengths of the filaments 46a, 46b extending proximally therefrom. The knot pusher 150 further includes a flexible shaft with column strength so that the knot 156 may be pushed through the access sheath 70. Slight traction is applied to the filaments 46a, 46b as the knot pusher 150 is advanced into and through the access sheath 70.

Figure 15B shows a final step where the knot 156 has been pushed through the access sheath 70 into contact with the proximal side of the annuloplasty ring 30, in the middle of the anterior segment as explained above. This operation is repeated to install between about 5-15 single knots in a compound knot to ensure that they will not come untied. The first two sutures could be placed in the same direction as a slip knot if desired, therefore allowing for some further tension adjustment, or in alternate directions to lock them. Knots are routinely tied through trocars in much the same way in laparoscopic surgery procedures in general surgery, orthopedics, Ob-Gyn, *etc.* The access sheath 70 is then retracted before or after severing the free ends of the filaments 46a, 46b close to the compound knot formed on the proximal side of the annuloplasty ring 30.

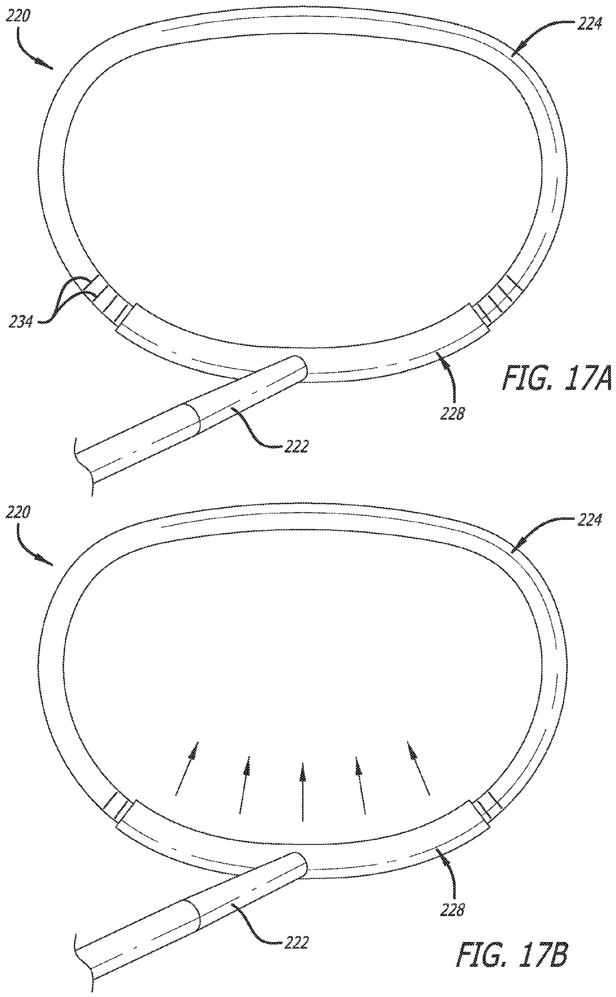

Figures 17A and 17B are plan views of a further alternative size-adjustable inner ring core 220 shown in initial and constricted states, respectively, and a distal end of a delivery system 222. The delivery system 222 may be connected to the ring size adjustment system 130 shown in Figure 14 so as to apply tension to one or both of a pair of adjustment filaments, as described above. The ring core 220 is typically surrounded by an outer suture permeable interface (not shown), and may be formed in a planar, semi-planar, or fully 3-dimensional saddle shape as described elsewhere.

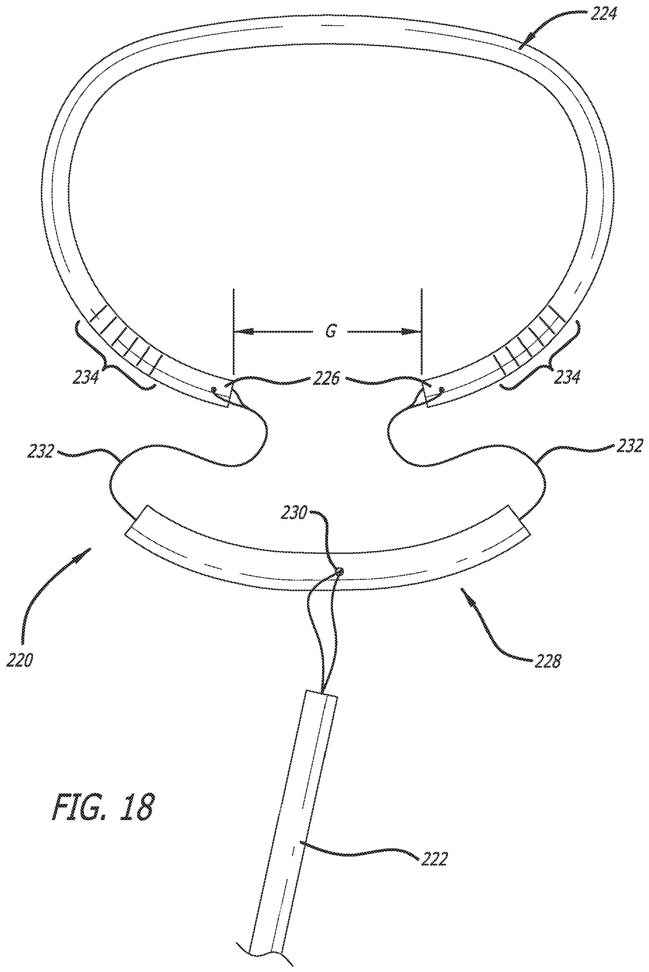

With reference first to the exploded view of Figure 18, the ring core 220 has an arcuate primary segment 224 with two free ends 226 on a posterior or more rounded side of the core. With reference back to Figure 1, the mitral annulus MA leaflets define scallops, and the posterior leaflet is divided into three scallops or cusps, sometimes identified as P1, P2, and P3. The two free ends 226 in the ring core 220 are located in the middle of the posterior segment of the mitral annulus, adjacent the P2 scallop of the posterior leaflet. The primary segment 224 may be seen as an anterior segment, as it spans the entire anterior aspect of the mitral annulus MA when implanted. The ring core 220 also has a sleeve-like secondary segment 228 that bridges the two free ends 226 of the primary segment 224. Though the secondary segment 228 spans only a portion of the posterior aspect of the mitral annulus MA when implanted, it may be seen as a posterior segment as it is centered on the posterior side.

The secondary segment 228 is arcuate, hollow and receives both of the two free ends 226 on either end so that the free ends may slide into the open ends of the secondary segment 228. As seen in Figure 18, the secondary segment 228 has a small aperture 230 located at its center on one side through which pass a pair of adjustment filaments 232. Each of the two adjustment filaments 232 has a free end secured to one of the free ends 226 of the primary ring core segment 224. In the illustrated embodiment, loops are formed in each free end of the filaments 232 which pass through holes in the free ends 226, though other anchoring solutions are also contemplated. The filaments 232 extend through the hollow interior of the secondary segment 228 and proximally out of the aperture 230, where they are received within a hollow interior of the delivery system 222. Typically, the delivery system 222 engages a proximal side of the ring core 220 such that the delivery system tube extends up out of the annulus, such as via the left atrium and out of an access port to an external location. For instance, the tube may pass through a small hole formed in the left atrial wall, and a hemostatic seal formed using a purse-string suture 136 as shown and described above with respect to Figure 14.

The primary segment 224 extends around at least 75% and more likely 90% of the periphery of the ring core 220, such that the two free ends 226 are separated across a gap G of between 8-12 mm. Annuloplasty rings are conventionally provided in 2 mm increments sizes measured across the major axis from 26-40 mm, though other measurement systems exist. The free ends 226 of the anterior/primary segment 224 are pulled and slide within the open ends of the posterior/secondary segment 228 by tension on the adjustment filaments 232 to reduce the gap G. The gap G may be between 30-40% of the distance across the major axis, which allows for a significant size reduction in the circumference of the ring, as will be explained.

First, to help the surgeon determine how much the ring constricts, hash marks 234 may be provided on one side of the ring core 220 and visible on external imaging, such as using fluoroscopy, such that a change in ring size can be observed from outside the body after the ring is installed and the heart is restarted. For example, the hash marks 234 seen in Figure 18 may be formed by lines of radiopaque material deposited on the ring core 220. Alternatively, the hash marks 234 may simply be score lines on the material of the ring core 220 which are visible using external imaging such as ultrasound. The hash marks 234 may be spaced apart 1 mm adjacent both free ends 226 of the primary segment 224 to make the extent of size adjustment easy to determine from outside the body. The terminal ends of the secondary segment 228 may also be configured to be similarly visible from external sensors so that the extent to which the primary and secondary segments 224, 228 overlap may be seen.

Figure 17A shows the ring core 220 in a neutral configuration in which the ring including the ring core has been implanted at the target annulus. Again, a "neutral" shape is calibrated so that the ring core 220 has a size that matches the desired size for an annuloplasty ring intended to be implanted at a particularly sized mitral annulus. The distal end of the delivery system 222 typically resembles a tube, as shown, through which the aforementioned adjustment filaments 232 extend. Once the ring is implanted, and a baseline blood flow detected through fluoroscopy or echocardiography, any regurgitation seen may be corrected by constructing the ring core 220.

Figure 17B shows the ring core 220 having been constricted to a certain extent by tensioning the adjustment filaments 232. As will be understood with reference to Figure 18, pulling the adjustment filaments 232 close the two free ends 226 inward into the gap G. Whereas initially four of the hash marks 234 on either side of the secondary segment 228 are visible in Figure 17A, after constriction only two are visible, indicating a 4 mm reduction in size of the gap G; i.e., 2 mm on each side. The 4 mm reduction in gap size does not mean a 4 mm reduction in the major axis of the ring core 220, merely a 4 mm reduction in the overall circumference of the ring. Moreover, because the gap G between the free ends 226 is located on the posterior side of the ring, movement from constriction of the ring core 220 occurs on the posterior side, as indicated by the inward arrows. This movement may be preferable for certain apologies, such as with congestive heart failure (CHF) in which the posterior side is often distended due to overall distention of the left ventricle. Of course, the free ends 226 may be located around the ring core 220 in other locations when indicated.

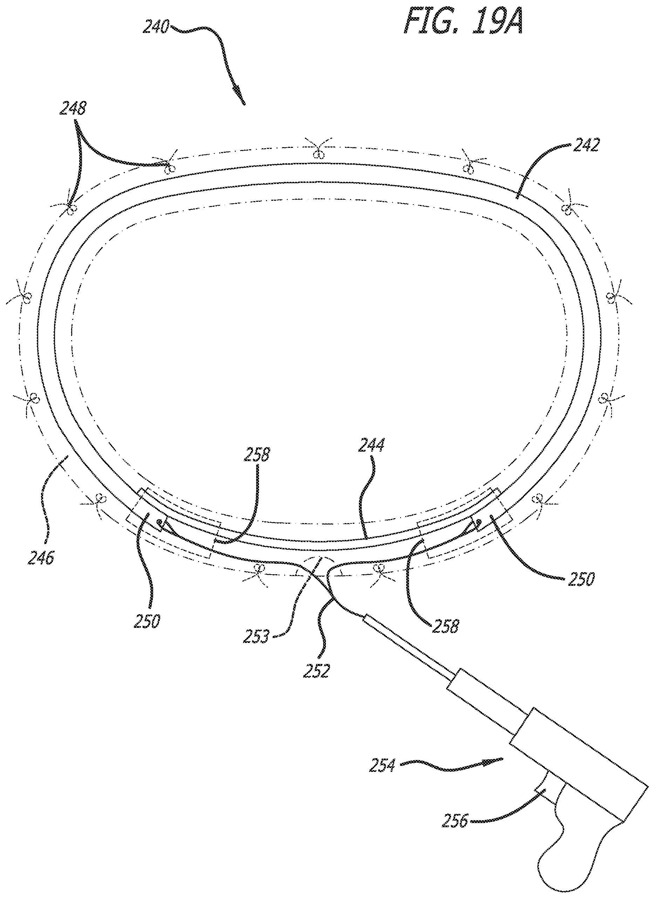

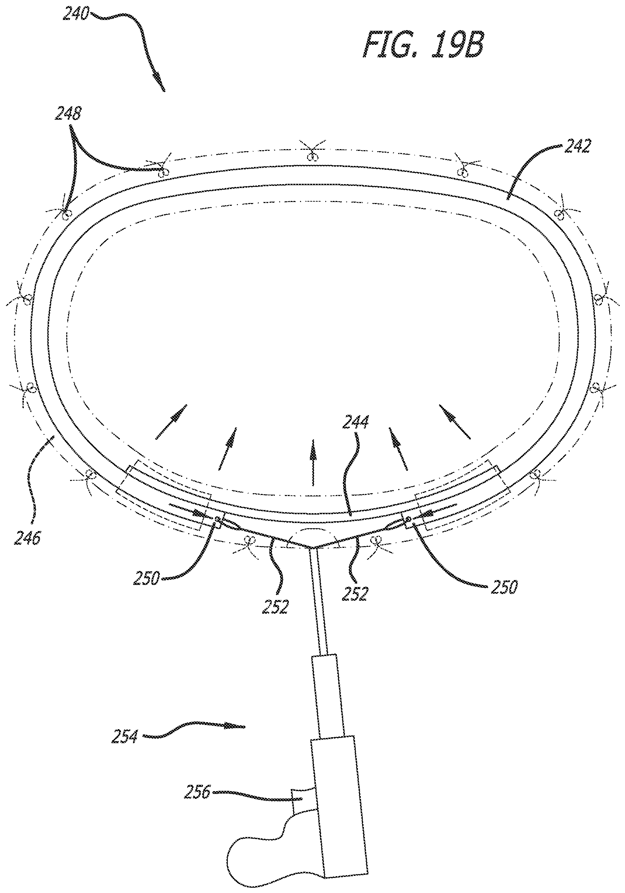

Figures 19A and 19B are plan views of another adjustable annuloplasty ring 240 shown in initial and constricted states, respectively, and a delivery system therefor. The annuloplasty ring 240 includes an inner ring core formed of an anterior or primary segment 242 and a posterior or secondary segment 244. In these illustrations, an outer suture permeable interface 246 is shown in dashed line. Specifically, the interface 246 comprises a fabric or silicone/fabric two closely surrounding the ring core. Anchoring suture knots 248 are also indicated to show how the annuloplasty ring 240 is typically secured to the native annulus. Of course, clips, staples or other such non-suture or knotless suture anchoring elements may be used. Again, the ring 240 may be formed in a planar, semi-planar, or fully 3-dimensional saddle shape as described elsewhere.

As with the embodiment shown in Figures 17-18, the primary segment 242 of the ring core extends around the majority of the circumference of the ring, preferably between about 75-90% of the entire circumference. The primary segment 242 has two free ends 250 which are spaced apart across a gap (not indicated), while the secondary segment 244 spans the gap and overlaps the primary segment two extent. In the illustrated embodiment, the secondary segment 244 has a thinner radio dimension than the primary segment 242 and lies against a radially inner face of the primary segment at the two free ends 250.

A pair of adjustment filaments 252 are respectively secured to the primary segment free ends 250, such as with a loop through holes, or via some other attachment as described elsewhere. The adjustment filaments 252 extend within the suture-permeable interface 246 to an exit opening 253, where they are received in a hollow distal end of a delivery system 254. In the illustrated embodiment, the delivery system 254 has a pistol-grip with a trigger 256 configured as an actuator to pull the filaments 252 and reduce the size of the annuloplasty ring 240. Sleeves 258, such as shrink-fit tubes, may surround the overlapping ends of the primary segment 242 and secondary segment 244 and maintain contact therebetween again, the size adjustment.

Figure 19B shows a size reduction step for the annuloplasty ring 240. By pulling the trigger 256, tension is applied to the adjustment filaments 252 which pulled both free ends 250 inward toward each other, as indicated by the circumferential arrows. This reduces the gap size between the two free ends 250, which slide along the outer face of the secondary segment 244. As explained above, the gap size may be reduced from between 8-12 mm to between 4-8 mm, or more. Again, the size adjustment is performed from outside the body under external visualization after the annuloplasty ring 240 has been anchored to the native annulus and the heart restarted.

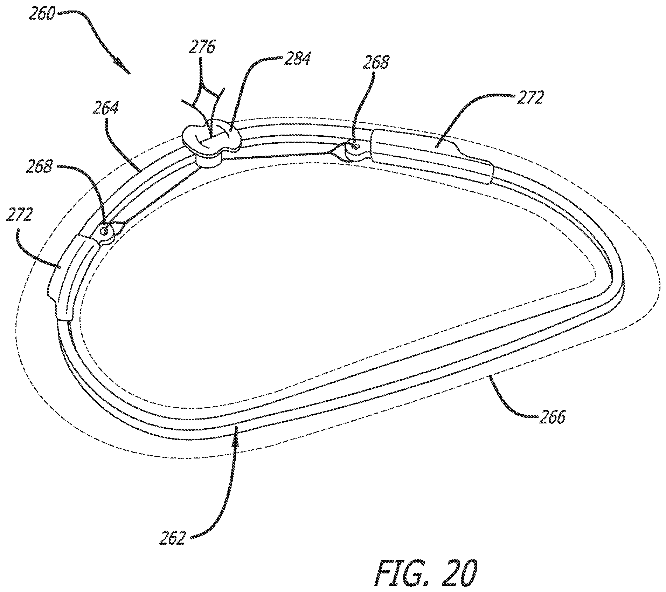

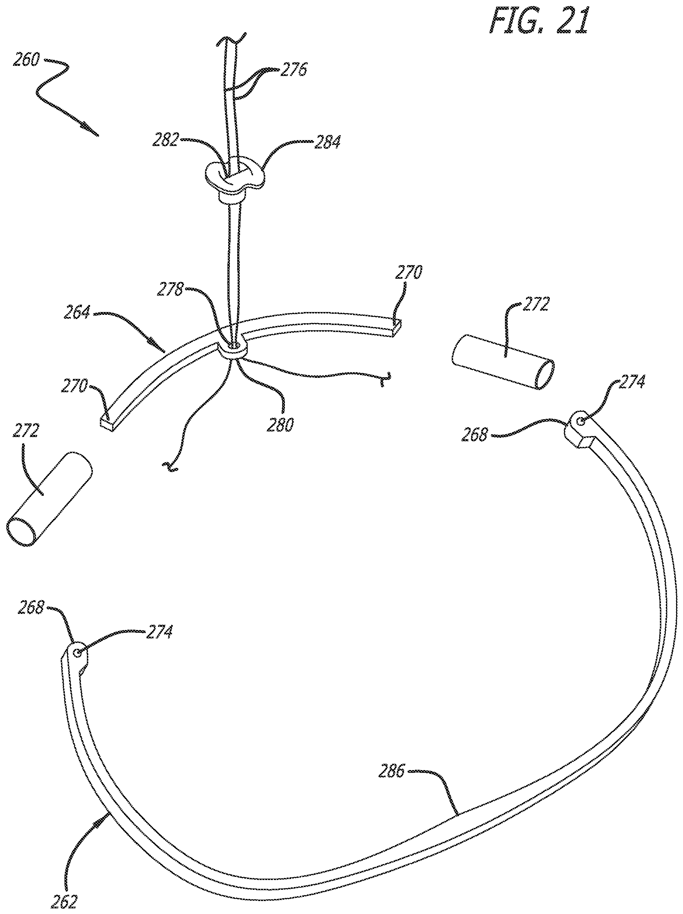

Figure 20 is a perspective assembled view of still another adjustable annuloplasty ring 260, and Figure 21 is an exploded view thereof. The ring core includes an anterior or primary segment 262 extending around a majority of the periphery or circumference of the ring 260, and a posterior or secondary segment 264 that overlaps the primary segment. The ring 260 also may include an outer suture-permeable interface 266 as described above. Again, the ring 260 may be formed in a planar, semi-planar, or fully 3-dimensional saddle shape as described elsewhere.

Figure 21 shows that the primary segment 262 has two free ends 268 which overlap two free ends 270 of the secondary segment 264. As with certain earlier embodiments, the primary segment 262 extends around the anterior aspect and most of the posterior aspect of the annuloplasty ring 260, with the secondary segment 264 closing a gap between the free ends 268 in the middle of the posterior side, as seen in Figure 20. In this embodiment, the secondary segment 264 overlaps the primary segment 262 on an outer face thereof, such that the two free ends 268 are located radially inside the secondary segment 264. A pair of tubular guide or alignment sleeves 272 may be added at the overlapping ends 268, 270 of the two segments. In one embodiment, the alignment sleeves 272 are shrink-fit tubes.

Each of the free ends 268 of the primary segment 262 have anchoring structure such as through holes 274 to which a pair of adjustment filaments 276 are secured. The filaments 276 extend distally through a delivery system tube (not shown) into proximity with the annuloplasty ring 260, and pass through an opening in the outer suture-permeable interface 266, and from there through an aperture 278 provided in a lug or grommet 280 projecting radially inward from the secondary segment 264. Once through the aperture 278, the filaments 276 diverge outwardly into connection with the free ends 268. Figure 20 shows the annuloplasty ring 260 having been constricted by applying tension to the adjustment filaments 276 which pull the free ends of the primary segment 262 toward each other along the secondary segment 264.

In one embodiment, the filaments 276 pass through a slit 282 formed in a knotless suture fastening clip 284 prior to reaching the annuloplasty ring 260. Once the proper adjustment to the size of the annuloplasty ring 260 has been attained, the surgeon will trim the ends of the filaments 276 with the clip 284 in close contact with the ring as indicated in Figure 20. The slit 282 is biased to clamp onto the filaments 276, thus fixing the size of the ring 260. Various knotless suture fastening clips known in the art may alternatively be used, such as the ones shown in U.S. Patent Nos. 5,520,702 and 9,498,202.

It should also be noted that the primary segment 262 has a generally radial cross-section section, with a thicker anterior section 286. Because the adjustment portion of the ring core is centered in the posterior side, the anterior side 286 undergoes little or no bending. Enlarging the cross-sectional size at interior section 286 ensures that the anterior side of the ring remains relatively unchanged.

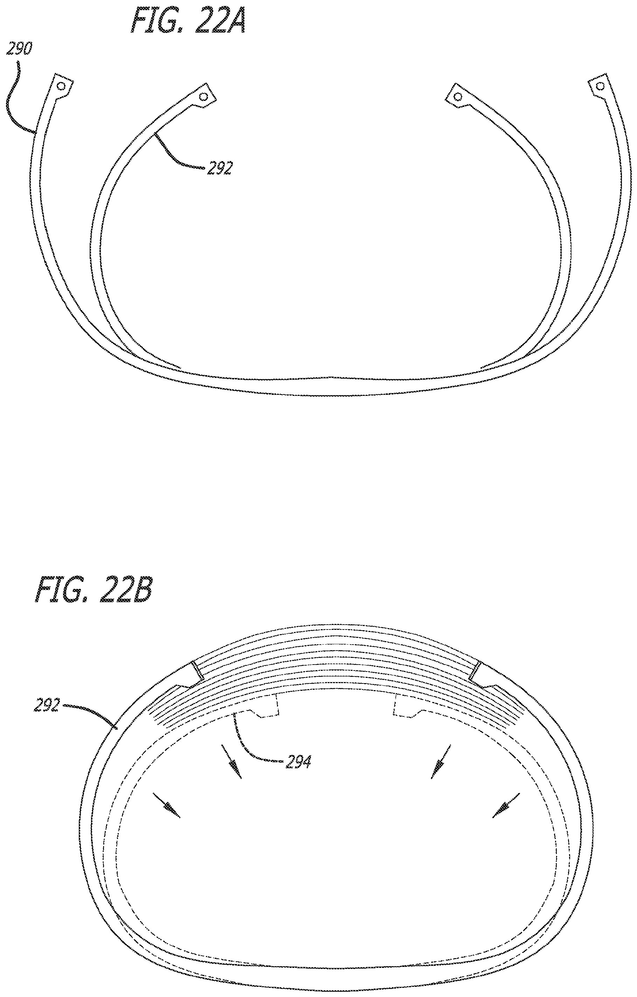

Figure 22A is a plan view of one segment 290 of the inner ring core of the adjustable annuloplasty ring of Figure 20, shown in both relaxed and neutral states, and Figure 22B schematically shows various constricted shapes that the segment may attain. The segment 290 resembles the primary segment 262 as seen in Figure 21, and circumscribes a majority of the periphery of the ring core. It should be understood that the segment 290 is representative of any of the primary segments of the ring cores described herein, and thus its properties are applicable to those other segments.

The segment 290 has a "relaxed" shape as shown by the wider representation, where the free ends are farther apart. The segment 290 further has a "neutral" shape as shown by the narrower representation 292, with the free ends closer together. Finally, Figure 22B shows a number of "constricted" shapes 294 indicated by a series of gradually smaller profiles, which may occur after implant if the ring is adjusted down in size.

When segment 290 is incorporated into one of the adjustable ring cores as described herein, the surgeon has the ability to both constrict the size of the ring while also being able to adjust the size upward from the neutral configuration. That is, it is relatively easy to attach tensioning filaments to one or more portions of the ring core for reducing the ring size. However, when the tissue filaments are relaxed, the ring segment may not easily expand beyond its initial neutral shape. The ring segment 290 may be formed of a resilient material with a relaxed shape that encourages expansion. That is, the neutral shape 292 is formed by pre-constricting the segment from the relaxed shape 290, which provides a natural outward bias. If the surgeon implants the ring and decides that it should be bigger, tension in the adjustment filaments is released so that the "spring back" bias encourages the ring segment to expand beyond the neutral shape 292 toward the relaxed shape 290. This "pre-loaded" outward bias in the segment from the neutral shape 292 to the relaxed shape 290 may be established by heat treating the material while in its relaxed shape. For example, Nitinol is a commonly used material in medical implants which can easily be heat treated to assume a particular shape.

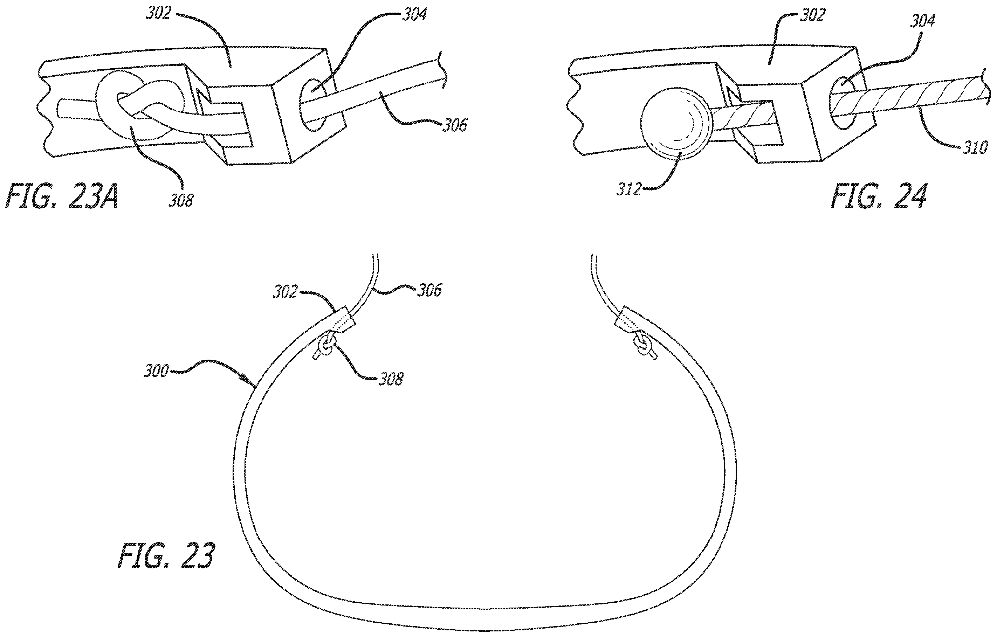

Figure 23 is a plan view of a primary segment 300 of an adjustable inner ring core with an enlarged free ends 302, and Figure 23A is an enlarged view of one free end showing an aperture 304 therethrough. Adjustment filaments 306 may be threaded through the aperture 304 and secured on the opposite side using one or more knots 308. This is perhaps the simplest way to assemble the adjustment filaments 306 to the ring segment 300. The filaments 306 may be conventional sutures or the like.

Figure 24 is a perspective view of a free end 302 of the segment 300 in Figure 23 showing an alternative attachment configuration. Namely, the adjustment filament 310 is shown as a braided cable, which may be polyethylene like suture material or a braided metallic cable. A bulbous head 312 is provided on the free end of the filament 310. The head 312 may be a spherical ball, as shown, or other such enlargement which is molded over or otherwise welded, soldered or adhered to the end of the cable 310. This means that the cable 310 must be fed in one direction through the aperture 304.

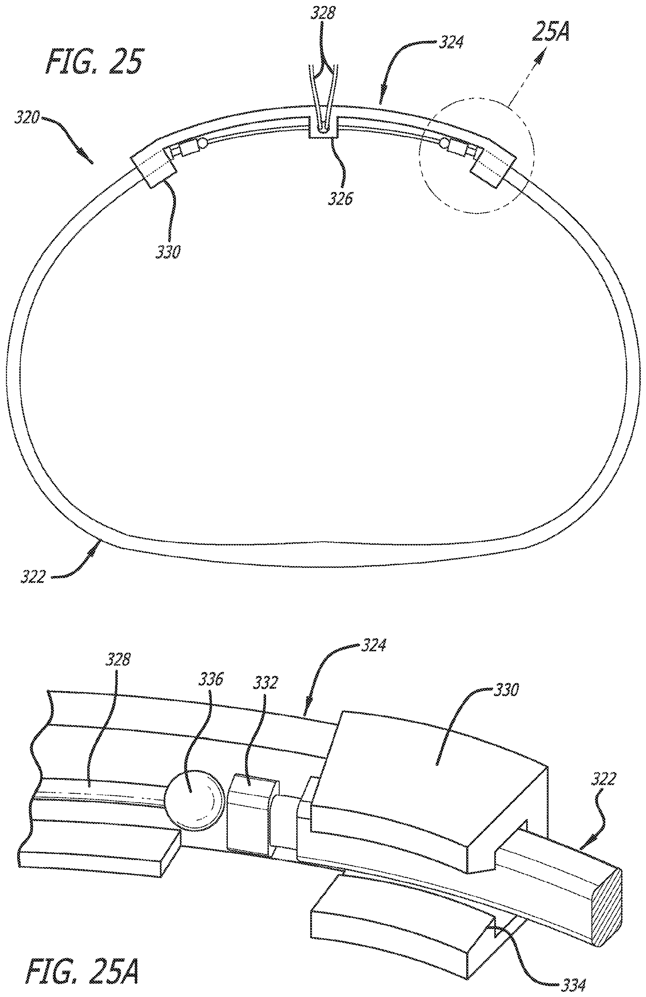

Figure 25 is a perspective view of another adjustable inner ring core 320 having a primary segment 322 and a secondary segment 324 which bridges a gap on a posterior side thereof. The secondary segment 324 has a central lug or grommet 326 through which a pair of adjustment filaments 320 extend. Size adjustment of the ring core 320 is similar to that described above with respect to the ring core 260, wherein the filaments 276 passed through a delivery system, through the grommet 280, and to the free ends of the primary segment 262.

Figure 25A is an enlargement showing a free end of the primary segment 322 passing through a guide member 330 formed at one free end of the secondary segment 324. The guide member 330 may have rails 334 which constrain the primary segment 322 as it passes therethrough so that the assembly remains aligned. The illustrated embodiment, the primary segment 322 has a rounded rectangular configuration which can be rotated 90° to fit between the rails 334 and into a larger space within the guide member 330.

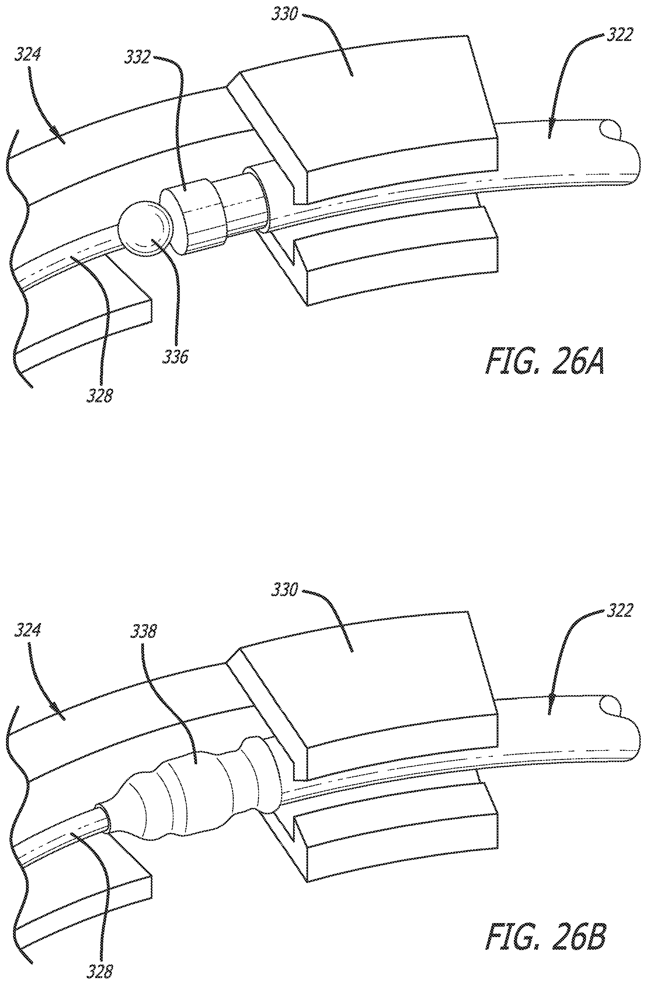

Each free end of the primary segment 322 terminates in a bulbous head 332 separated from the remainder of the segment by a narrowed neck. In Figure 25A the bulbous head 332 has a rounded rectangular configuration, while in Figure 26A the bulbous head 332 is cylindrical. At the same time, each of the adjustment filaments 328 terminates in a bulbous (e.g., spherical) head 336. The adjustment filaments 328 connect to the respective free ends 332 of the primary segment 322 via a shrink tubing 338, as seen in Figure 26B. It will thus be apparent that any non-linear shaped free end may be utilized and connected to the adjustment filaments using shrink fit tubing.

While the foregoing is a complete description of the preferred examples, various alternatives, modifications, and equivalents may be used. Moreover, it will be obvious that certain other modifications may be practiced.

## Claims

1. An annuloplasty ring and shape adjustment system, comprising:
a three-dimensional annuloplasty ring (30) defining a continuous peripheral shape around a central aperture, the annuloplasty ring (30) having an inner core (40; 80) formed of an elastic-plastic material and a suture-permeable interface surrounding the inner core (40; 80) and extending around the peripheral shape, the inner core (40; 80) having an arcuate anterior segment (42; 82) that overlaps an arcuate posterior segment (44; 84) on opposite sides, the peripheral shape being a rounded D-shape in plan view wherein both the anterior segment (42; 82) and posterior segment (44; 84) rise up from the opposite sides to form a saddle shape, and the anterior and posterior segments (42, 44; 82, 84) define a ratchet mechanism at overlapped regions on opposite sides such that a position of the anterior segment (42; 82) relative to the posterior segment (44; 84) may be changed and the peripheral shape of the annuloplasty ring (30) may be adjusted;
a pair of adjustment filaments (46a, 46b) adapted to pass through passages in one of the segments (42, 44; 82, 84) of the inner core (40; 80), the filaments (46a, 46b) being connected to and displace the other segment on opposite sides to adjust the peripheral shape of the annuloplasty ring (30);
a plurality of anchoring sutures adapted to pass through the interface and secure the annuloplasty ring (30) to a native heart valve annulus; and
a shape adjusting mechanism including an access sheath (70) having a length sufficient to extend from the native heart valve annulus through an access incision in the heart and out of the body, the pair of adjustment filaments (46a, 46b) passing through the access sheath (70), the shape adjusting mechanism having a pair of tension adjusters (138a, 138b) and each of the adjustment filaments (46a, 46b) passing separately through an associated one of the tension adjusters (138a, 138b) to enable separate displacement of the adjustment filaments (46a, 46b) relative to the annuloplasty ring (30), the access sheath (70) having sufficient column strength such that tension on either of the adjustment filaments (46a, 46b) caused by the associated tension adjuster (138a, 138b) adjusts the peripheral shape of the annuloplasty ring (30).

2. The system of claim 1, wherein the passages are formed in the anterior segment (42; 82) and open to two closely-spaced entry holes (60) on an atrial side of a midsection (50; 85) thereof, the passages being configured such that the adjustment filaments (46a, 46b) diverge from the entry holes (60) and emerge on a radially outward side of the midsection (50; 85), wherein secondary passages are formed in the anterior segment (42; 82) such that the adjustment filaments (46a, 46b) extend into inner channels (55) formed by side sections (64; 86) of the anterior segment (42; 82), wherein free ends of the posterior segment (44; 84) overlap the anterior segment (42; 82) in the inner channels (55), and the adjustment filaments (46a, 46b) attach to the free ends of the posterior segment (44; 84).

3. The system of claim 2, further including a biasing spring (48) positioned within each of the inner channels (55) that applies a compressive force to both the anterior segment (42; 82) and the posterior segment (44; 84) tending to force them apart.

4. The system of any of claims 1 to 3, wherein the ratchet mechanism includes teeth (66) on one segment engaging a detent (58) on the other segment.

5. The system of claim 4, wherein each side section (52) of the anterior segment (42) includes the detent (58) projecting into inner channels formed by side sections (52) of the anterior segment (42), and the posterior segment (44) includes a midsection (62) and two side sections (64) ending at the free ends, wherein each of the side sections (64) of the posterior segment (44) has a series of the teeth (66) which the detents (58) engage in each of the inner channels (55), the detents (58) and teeth (66) being arranged to prevent the side section (64) of the posterior segment (44) on each side from retracting from within the inner channels (55).

6. The system of any of claims 1 to 3, wherein the ratchet mechanism includes undulating bumps (92) on one segment engaging mating undulating bumps (94) on the other segment; in particular further including a compression sleeve (96) surrounding each of the overlapped regions on opposite sides of the anterior and posterior segments (42, 44; 82, 84).

7. The system of any of claims 1 to 6, further including a hemostatic valve positioned at a proximal end of the access sheath (70), the system also comprising a purse-string suture (136) adapted to seal an access incision into the left atrium around the access sheath (70); and/or wherein each of the tension adjusters (138a, 138b) has a rotating dial to adjust the tension on the associated adjustment filaments (46a, 46b), and a gauge (140) calibrated to indicate distance that the associated adjustment filament (46a, 46b) is displaced.

8. A method of simulating implantation of an annuloplasty ring (30) in a simulated patient at a native heart valve annulus and adjusting a peripheral shape of the annuloplasty ring (30), comprising:
stopping a simulated patient's heart and providing an access pathway to a native heart valve annulus though a body to the heart, including an access incision in the heart leading to a heart valve annulus;
looping a plurality of anchoring sutures through the heart valve annulus at spaced locations and retracting free ends of the anchoring sutures out of the body;
passing one end of each of the pairs of free ends of the anchoring sutures through a peripheral suture-permeable interface of an annuloplasty ring (30), the annuloplasty ring (30) including an inner core (40; 80) defining a continuous peripheral shape around a central aperture and the interface closely surrounding the inner core (40; 80) and having a matching shape, the inner core (40; 80) having an arcuate anterior segment (42; 82) that overlaps an arcuate posterior segment (44; 84)con opposite sides, the peripheral shape being a rounded D-shape in plan view wherein both the anterior segment (42; 82) and posterior segment (44; 84) rise up from the opposite sides to form a saddle shape, and the anterior and posterior segments (42, 44; 82, 84) define a ratchet mechanism at overlapped regions on opposite sides such that a position of the anterior segment (42, 82) relative to the posterior segment (44, 84) may be changed and the peripheral shape of the annuloplasty ring (30) may be adjusted, the annuloplasty ring (30) further including a pair of adjustment filaments (46a, 46b) adapted to pass through passages in one segment of the inner core (40; 80), the filaments (46a, 46b) being connected to and displace the other segment on opposite sides to adjust the peripheral shape of the annuloplasty ring (30);
passing both adjustment filaments (46a, 46b) through an access sheath (70), the access sheath (70) and adjustment filaments (46a, 46b) having sufficient lengths to extend out of the body, each of the adjustment filaments (46a, 46b) passing separately through a tension adjuster (138a, 138b) to enable separate displacement of the adjustment filaments (46a, 46b) relative to the annuloplasty ring (30);
advancing the annuloplasty ring (30) to the heart valve annulus using the access sheath (70) along the anchoring sutures;
tying off the anchoring sutures on a proximal side of the annuloplasty ring (30);
sealing the access incision around the access sheath (70);
restarting the patient's heart;
externally visualizing blood flow through the heart valve annulus;
adjusting the tension on one or both of the adjustment filaments (46a, 46b) until regurgitation through the heart valve annulus is minimized;
severing the adjustment filaments (46a, 46b) on a proximal side of the annuloplasty ring (30); and
withdrawing the adjustment filaments (46a, 46b) and access sheath (70) from the body and closing up the access pathway.

9. The method of claim 8, wherein the passages are formed in the anterior segment (42; 82) and open to two closely-spaced entry holes on an atrial side of a midsection (50; 85) thereof, the passages being configured such that the adjustment filaments (46a, 46b) diverge from the entry holes (60) and emerge on a radially outward side of the midsection (50; 85), wherein secondary passages are formed in the anterior segment (42; 82) such that the adjustment filaments (46a, 46b) extend into inner channels (55) formed by side sections (64; 86) of the anterior segment (42; 82), wherein free ends of the posterior segment (44; 84) overlap the anterior segment (42; 82) in the inner channels (55), and the adjustment filaments (46a, 46b) attach to the free ends of the posterior segment (44; 84); in particular further including a biasing spring positioned within each of the inner channels (55) that applies a compressive force to both the anterior segment (42; 82) and the posterior segment (44; 84) tending to force them apart.

10. The method of any of claims 8 or 9, wherein the ratchet mechanism includes teeth on one segment engaging a detent on the other segment, and wherein each side section of the anterior segment includes the detent projecting into each of the inner channels (55), and the posterior segment (44; 84) includes a midsection and two side sections ending at the free ends, wherein each of the side sections of the posterior segment (44; 84) has a series of the teeth which the detents engage in each of the inner channels (55), the detents and teeth being arranged to prevent the side section of the posterior segment (44; 84) on each side from retracting from within the inner channels (55).

11. The method of any of claims 8 or 9, wherein the ratchet mechanism includes teeth (66) on one segment engaging a detent (58) on the other segment.

12. The method of any of claims 8 or 9, wherein the ratchet mechanism includes undulating bumps (92) on one segment engaging mating undulating bumps on the other segment; in particular further including a compression sleeve surrounding each of the overlapped regions on opposite sides of the anterior and posterior segments.

13. The method of any of claims 8 to 12, further including a hemostatic valve positioned at a proximal end of the access sheath (70), the system also comprising a purse-string suture (136) adapted to seal an access incision into the left atrium around the access sheath (70).

14. The method of any of claims 8 to 13, wherein each of the tension adjusters (138a, 138b) has a rotating dial to adjust the tension on the associated adjustment filaments (46a, 46b), and a gauge (140) calibrated to indicate distance that the associated adjustment filament (46a, 46b) is displaced.

15. The method of any of claims 8 to 14, further including using a knot pusher (150) to advance single knots (156) between the adjustment filaments (46a, 46b) through the access sheath (70) to create a compound knot between the adjustment filaments (46a, 46b) on a proximal side of the annuloplasty ring (30) prior to severing the adjustment filaments (46a, 46b).

## Patentansprüche

1. Anuloplastie-Ring- und Formeinstell-System, umfassend:
einen dreidimensionalen Annuloplastie-Ring (30), der eine kontinuierliche periphere Form um eine zentrale Öffnung definiert, wobei der Annuloplastie-Ring (30) einen inneren Kern (40; 80), der aus einem elastisch-plastischen Material gebildet ist, und eine für Nahtmaterial durchlässige Grenzfläche aufweist, die den inneren Kern (40; 80) umgibt und sich um die periphere Form erstreckt, wobei der innere Kern (40; 80) ein bogenförmiges anteriores Segment (42; 82) aufweist, das ein bogenförmiges posteriores Segment (44; 84) auf gegenüberliegenden Seiten überlappt, wobei die periphere Form in der Draufsicht eine abgerundete D-Form ist, wobei sowohl das anteriore Segment (42; 82) als auch das posteriore Segment (44; 84) von den gegenüberliegenden Seiten aufragen, um eine Sattelform zu bilden, und das anteriore und das posteriore Segment (42, 44; 82, 84) an überlappenden Bereichen auf gegenüberliegenden Seiten einen Ratschenmechanismus definieren, so dass eine Position des anterioren Segments (42; 82) relativ zum posterioren Segment (44; 84) verändert werden kann und die periphere Form des Annuloplastie-Rings (30) eingestellt werden kann;
ein Paar von Einstellfilamenten (46a, 46b), die dazu eingerichtet sind, durch Durchgänge in einem der Segmente (42, 44; 82, 84) des inneren Kerns (40; 80) zu verlaufen, wobei die Filamente (46a, 46b) mit dem anderen Segment auf gegenüberliegenden Seiten verbunden sind und dieses verschieben, um die periphere Form des Anuloplastie-Rings (30) einzustellen;
eine Vielzahl von Verankerungsnähfäden, die dazu eingerichtet sind, durch die Grenzfläche zu verlaufen und den Anuloplastie-Ring (30) an einem nativen Herzklappenanulus zu befestigen; und
eine Formeinstelleinrichtung, die eine Zugangshülle (70) mit einer Länge aufweist, die ausreicht, um sich vom nativen Herzklappenanulus durch einen Zugangsschnitt im Herzen und aus dem Körper heraus zu erstrecken, wobei das Paar von Einstellfilamenten (46a, 46b) durch die Zugangshülle (70) verläuft, die Formeinstelleinrichtung ein Paar von Spannungseinstellern (138a, 138b) aufweist und jedes der Einstellfilamente (46a, 46b) separat durch einen zugehörigen Spannungseinsteller (138a, 138b) verläuft, um eine separate Verschiebung der Einstellfilamente (46a, 46b) relativ zu dem Annuloplastie-Ring (30) zu ermöglichen, wobei die Zugangshülle (70) eine ausreichende Knickfestigkeit aufweist, so dass eine Spannung an einem der Einstellfilamente (46a, 46b), die durch den zugehörigen Spannungseinsteller (138a, 138b) bewirkt wird, die periphere Form des Annuloplastie-Rings (30) einstellt.

2. System nach Anspruch 1, wobei die Durchgänge in dem anterioren Segment (42; 82) ausgebildet sind und sich zu zwei eng beabstandeten Eintrittslöchern (60) auf einer atrialen Seite eines Mittelabschnitts (50; 85) davon öffnen, wobei die Durchgänge so konfiguriert sind, dass die Einstellfilamente (46a, 46b) von den Eintrittslöchern (60) divergieren und an einer radial äußeren Seite des Mittelabschnitts (50; 85) austreten, wobei sekundäre Durchgänge in dem anterioren Segment (42; 82) ausgebildet sind, so dass sich die Einstellfilamente (46a, 46b) in innere Kanäle (55) erstrecken, die von Seitenabschnitten (64; 86) des anterioren Segments (42; 82) gebildet werden, wobei freie Enden des posterioren Segments (44; 84) das anteriore Segment (42; 82) in den inneren Kanälen (55) überlappen, und die Einstellfilamente (46a, 46b) an den freien Enden des posterioren Segments (44; 84) angebracht sind.

3. System nach Anspruch 2, das ferner eine Vorspannfeder (48) enthält, die in jedem der inneren Kanäle (55) angeordnet ist und eine komprimierende Kraft sowohl auf das anteriore Segment (42; 82) als auch auf das posteriore Segment (44; 84) ausübt, die diese auseinander zu drücken sucht.

4. System nach einem der Ansprüche 1 bis 3, wobei der Ratschenmechanismus Zähne (66) an einem Segment aufweist, die mit einer Rastnase (58) an dem anderen Segment in Eingriff kommen.

5. System nach Anspruch 4, wobei jeder Seitenabschnitt (52) des anterioren Segments (42) die Rastnase (58) aufweist, die in die von den Seitenabschnitten (52) des anterioren Segments (42) gebildeten inneren Kanäle ragt, und das posteriore Segment (44) einen Mittelabschnitt (62) und zwei an den freien Enden endende Seitenabschnitte (64) aufweist, wobei jeder der Seitenabschnitte (64) des posterioren Segments (44) eine Reihe von Zähnen (66) aufweist, in die die Rastnasen (58) in jedem der inneren Kanäle (55) eingreifen, wobei die Rastnasen (58) und die Zähne (66) so angeordnet sind, dass sie verhindern, dass sich der Seitenabschnitt (64) des posterioren Segments (44) auf jeder Seite aus dem Inneren der inneren Kanäle (55) zurückzieht.

6. System nach einem der Ansprüche 1 bis 3, wobei der Ratschenmechanismus wellenförmige Erhebungen (92) auf einem Segment aufweist, die mit passenden wellenförmigen Erhebungen (94) auf dem anderen Segment in Eingriff kommen; insbesondere ferner umfassend eine Kompressionshülle (96), die jeden der überlappenden Bereiche auf gegenüberliegenden Seiten des anterioren und des posterioren Segments (42, 44; 82, 84) umgibt.

7. System nach einem der Ansprüche 1 bis 6, das ferner ein hämostatisches Ventil enthält, das an einem proximalen Ende der Zugangshülle (70) angeordnet ist, wobei das System auch einen Raffnaht-Faden (136) umfasst, der dazu eingerichtet ist, einen Zugangsschnitt in das linke Atrium um die Zugangshülle (70) herum abzudichten; und/oder wobei jeder der Spannungseinsteller (138a, 138b) eine drehbare Einstellscheibe aufweist, um die Spannung an den zugehörigen Einstellfilamenten (46a, 46b) einzustellen, und eine Messeinrichtung (140), die so kalibriert ist, dass sie die Distanz angibt, um die das zugehörige Einstellfilament (46a, 46b) verschoben ist.

8. Verfahren zum Simulieren der Implantation eines Annuloplastie-Rings (30) in einem simulierten Patienten an einem nativen Herzklappenanulus und zum Einstellen einer peripheren Form des Annuloplastie-Rings (30), umfassend:
Anhalten des Herzens eines simulierten Patienten und Bereitstellen eines Zugangsweges zu einem nativen Herzklappenanulus durch einen Körper zum Herzen, einschließlich eines Zugangsschnittes im Herzen, der zu einem Herzklappenanulus führt;
Durchschleifen einer Vielzahl von Verankerungsnähfäden durch den Herzklappenanulus an beabstandeten Stellen und Zurückziehen der freien Enden der Verankerungsnähfäden aus dem Körper;
Durchführen eines Endes jedes der Paare freier Enden der Verankerungsnähte durch eine periphere nahtdurchlässige Grenzfläche eines Anuloplastie-Rings (30), wobei der Anuloplastie-Ring (30) einen inneren Kern (40; 80) aufweist, der eine kontinuierliche periphere Form um eine zentrale Öffnung definiert, und die Grenzfläche den inneren Kern (40; 80) eng umgibt und eine passende Form aufweist, wobei der innere Kern (40; 80) ein bogenförmiges anteriores Segment (42; 82) aufweist, das ein bogenförmiges posteriores Segment (44; 84) auf gegenüberliegenden Seiten überlappt, wobei die periphere Form in der Draufsicht eine abgerundete D-Form ist, wobei sowohl das anteriore Segment (42; 82) als auch das posteriore Segment (44; 84) von den gegenüberliegenden Seiten aufragen, um eine Sattelform zu bilden, und das anteriore und das posteriore Segment (42, 44; 82, 84) einen Ratschenmechanismus an überlappenden Bereichen auf gegenüberliegenden Seiten definieren, so dass eine Position des anterioren Segments (42, 82) relativ zum posterioren Segment (44, 84) verändert werden kann und die periphere Form des Annuloplastie-Rings (30) eingestellt werden kann, wobei der Annuloplastie-Ring (30) ferner ein Paar von Einstellfilamenten (46a, 46b) aufweist, die dazu eingerichtet sind, durch Durchgänge in einem Segment des inneren Kerns (40; 80) zu verlaufen, wobei die Filamente (46a, 46b) mit dem anderen Segment auf gegenüberliegenden Seiten verbunden sind und dieses verschieben, um die periphere Form des Annuloplastie-Rings (30) einzustellen;
Durchführen beider Einstellfilamente (46a, 46b) durch eine Zugangshülle (70), wobei die Zugangshülle (70) und die Einstellfilamente (46a, 46b) eine ausreichende Länge aufweisen, um sich aus dem Körper heraus zu erstrecken, wobei jedes der Einstellfilamente (46a, 46b) separat durch einen Spannungseinsteller (138a, 138b) verläuft, um eine separate Verschiebung der Einstellfilamente (46a, 46b) relativ zu dem Annuloplastie-Ring (30) zu ermöglichen;
Vorschieben des Annuloplastie-Rings (30) zum Herzklappenanulus unter Verwendung der Zugangshülle (70) entlang der Verankerungsnähfäden;
Abbinden der Verankerungsnähte an einer proximalen Seite des Anuloplastie-Rings (30);
Abdichten des Zugangsschnittes um die Zugangshülle (70);
Wiederherstellung der Herzfunktion des Patienten;
externes Visualisieren des Blutflusses durch den Herzklappenanulus;
Einstellen der Spannung an einem oder beiden Einstellfilamenten (46a, 46b), bis die Regurgitation durch den Herzklappenanulus minimiert ist;
Durchtrennen der Einstellfilamente (46a, 46b) an einer proximalen Seite des Anuloplastie-Rings (30); und
Zurückziehen der Einstellfilamente (46a, 46b) und der Zugangshülle (70) aus dem Körper und Verschließen des Zugangsweges.

9. Verfahren nach Anspruch 8, wobei die Durchgänge in dem anterioren Segment (42; 82) ausgebildet sind und sich zu zwei eng beabstandeten Eintrittslöchern auf einer atrialen Seite eines Mittelabschnitts (50; 85) davon öffnen, wobei die Durchgänge so konfiguriert sind, dass die Einstellfilamente (46a, 46b) von den Eintrittslöchern (60) divergieren und an einer radial äußeren Seite des Mittelabschnitts (50) austreten; 85) austreten, wobei sekundäre Durchgänge in dem anterioren Segment (42; 82) ausgebildet sind, so dass sich die Einstellfilamente (46a, 46b) in innere Kanäle (55) erstrecken, die von Seitenabschnitten (64; 86) des anterioren Segments (42; 82) gebildet werden, wobei freie Enden des posterioren Segments (44; 84) das anteriore Segment (42; 82) in den inneren Kanälen (55) überlappen, und die Einstellfilamente (46a, 46b) an den freien Enden des posterioren Segments (44; 84) angebracht sind; insbesondere ferner eine Vorspannfeder enthaltend, die in jedem der inneren Kanäle (55) angeordnet ist und eine komprimierende Kraft sowohl auf das anteriore Segment (42; 82) als auch auf das posteriore Segment (44; 84) ausübt, die diese auseinander zu drücken sucht.

10. Verfahren nach einem der Ansprüche 8 oder 9, wobei der Ratschenmechanismus Zähne an einem Segment aufweist, die mit einer Rastnase an dem anderen Segment in Eingriff kommen, und wobei jeder Seitenabschnitt des anterioren Segments die Rastnase aufweist, die jeweils in die inneren Kanäle (55) ragt, und das posteriore Segment (44; 84) einen mittleren Abschnitt und zwei an den freien Enden endende Seitenabschnitte aufweist, wobei jeder der Seitenabschnitte des posterioren Segments (44; 84) eine Reihe von Zähnen aufweist, in die die Rastnasen in jedem der inneren Kanäle (55) eingreifen, wobei die Rastnasen und Zähne so angeordnet sind, dass sie verhindern, dass sich der Seitenabschnitt des posterioren Segments (44; 84) auf jeder Seite aus dem Inneren der inneren Kanäle (55) zurückzieht.

11. Verfahren nach einem der Ansprüche 8 oder 9, wobei der Ratschenmechanismus Zähne (66) an einem Segment aufweist, die mit einer Rastnase (58) an dem anderen Segment in Eingriff kommen.

12. Verfahren nach einem der Ansprüche 8 oder 9, wobei der Ratschenmechanismus wellenförmige Erhebungen (92) auf einem Segment aufweist, die mit passenden wellenförmigen Erhebungen auf dem anderen Segment in Eingriff kommen; insbesondere ferner umfassend eine Kompressionshülle, die jeden der überlappenden Bereiche auf gegenüberliegenden Seiten des anterioren und des posterioren Segments umgibt.

13. Verfahren nach einem der Ansprüche 8 bis 12, das ferner ein hämostatisches Ventil enthält, das an einem proximalen Ende der Zugangshülle (70) angeordnet ist, wobei das System auch einen Raffnaht-Faden (136) umfasst, der dazu eingerichtet ist, einen Zugangsschnitt in das linke Atrium um die Zugangshülle (70) herum abzudichten.

14. Verfahren nach einem der Ansprüche 8 bis 13, wobei jeder der Spannungseinsteller (138a, 138b) eine drehbare Einstellscheibe aufweist, um die Spannung an den zugehörigen Einstellfilamenten (46a, 46b) einzustellen, und eine Messeinrichtung (140), die so kalibriert ist, dass sie die Distanz angibt, um die das zugehörige Einstellfilament (46a, 46b) verschoben ist.

15. Verfahren nach einem der Ansprüche 8 bis 14, das ferner die Verwendung eines Knotenschiebers (150) umfasst, um einzelne Knoten (156) zwischen den Einstellfilamenten (46a, 46b) durch die Zugangshülle (70) vorzuschieben, um einen Verbundknoten zwischen den Einstellfilamenten (46a, 46b) auf einer proximalen Seite des Annuloplastie-Rings (30) herzustellen, bevor die Einstellfilamente (46a, 46b) durchtrennt werden.

## Revendications

1. Système d'anneau d'annuloplastie et d'ajustement de forme, comprenant :
un anneau d'annuloplastie (30) tridimensionnel définissant une forme périphérique continue autour d'une ouverture centrale, l'anneau d'annuloplastie (30) présentant un noyau interne (40 ; 80) formé d'un matériau plastique élastique et d'une interface perméable à la suture entourant le noyau interne (40 ; 80) et s'étendant autour de la forme périphérique, le noyau interne (40 ; 80) présentant un segment antérieur (42 ; 82) arqué qui chevauche un segment postérieur (44 ; 84) arqué sur des côtés opposés, la forme périphérique étant une forme arrondie en D dans une vue en plan, à la fois le segment antérieur (42 ; 82) et le segment postérieur (44 ; 84) s'élevant à partir des côtés opposés afin de former une forme de selle et les segments antérieur et postérieur (42, 44 ; 82, 84) définissant un mécanisme à cliquet au niveau de régions chevauchées sur des côtés opposés de telle sorte qu'une position du segment antérieur (42 ; 82) par rapport au segment postérieur (44 ; 84) peut être modifiée et la forme périphérique de l'anneau d'annuloplastie (30) peut être ajustée ;
une paire de filaments (46a, 46b) d'ajustement conçus pour passer à travers des passages dans l'un des segments (42, 44 ; 82, 84) du noyau interne (40 ; 80), les filaments (46a, 46b) étant reliés à l'autre segment et déplaçant celui-ci sur des côtés opposés afin d'ajuster la forme périphérique de l'anneau d'annuloplastie (30) ;
une pluralité de sutures d'ancrage conçues pour passer à travers l'interface et fixer l'anneau d'annuloplastie (30) à un anneau de valvule cardiaque native ; et
un mécanisme d'ajustement de forme comprenant une gaine d'accès (70) présentant une longueur suffisante pour s'étendre à partir de l'anneau de valvule cardiaque native à travers une incision d'accès dans le cœur et hors du corps, la paire de filaments (46a, 46b) d'ajustement passant à travers la gaine d'accès (70), le mécanisme d'ajustement de forme présentant une paire de dispositifs d'ajustement de tension (138a, 138b) et chacun des filaments (46a, 46b) d'ajustement passant séparément à travers un dispositif d'ajustement associé parmi les dispositifs d'ajustement de tension (138a, 138b) afin de permettre un déplacement séparé des filaments (46a, 46b) d'ajustement par rapport à l'anneau d'annuloplastie (30), la gaine d'accès (70) présentant une résistance en colonne suffisante de telle sorte qu'une tension sur l'un ou l'autre des filaments (46a, 46b) d'ajustement provoquée par le dispositif d'ajustement de tension (138a, 138b) associé ajuste la forme périphérique de l'anneau d'annuloplastie (30).

2. Système selon la revendication 1, les passages étant formés dans le segment antérieur (42 ; 82) et ouverts vers deux trous d'entrée (60) étroitement écartés sur un côté auriculaire d'une section médiane (50 ; 85) correspondante, les passages étant conçus de telle sorte que les filaments (46a, 46b) d'ajustement divergent à partir des trous d'entrée (60) et émergent sur un côté radialement vers l'extérieur de la section médiane (50 ; 85), des passages secondaires étant formés dans le segment antérieur (42 ; 82) de telle sorte que les filaments (46a, 46b) d'ajustement s'étendent dans des canaux internes (55) formés par des sections latérales (64 ; 86) du segment antérieur (42 ; 82), des extrémités libres du segment postérieur (44 ; 84) chevauchant le segment antérieur (42 ; 82) dans les canaux internes (55) et les filaments (46a, 46b) d'ajustement s'attachant aux extrémités libres du segment postérieur (44 ; 84).

3. Système selon la revendication 2, comprenant en outre un ressort de sollicitation (48) positionné à l'intérieur de chacun des canaux internes (55) qui applique une force de compression à la fois au segment antérieur (42 ; 82) et au segment postérieur (44 ; 84), tendant à forcer leur écartement.

4. Système selon l'une quelconque des revendications 1 à 3, le mécanisme à cliquet comprenant des dents (66) sur un segment venant en prise avec un cran d'arrêt (58) sur l'autre segment.

5. Système selon la revendication 4, chaque section latérale (52) du segment antérieur (42) comprenant le cran d'arrêt (58) faisant saillie dans des canaux internes formés par des sections latérales (52) du segment antérieur (42) et le segment postérieur (44) comprenant une section médiane (62) et deux sections latérales (64) se terminant au niveau des extrémités libres, chacune des sections latérales (64) du segment postérieur (44) présentant une série des dents (66) avec lesquelles les crans d'arrêt (58) viennent en prise dans chacun des canaux internes (55), les crans d'arrêt (58) et les dents (66) étant agencés pour empêcher la rétraction de la section latérale (64) du segment postérieur (44) sur chaque côté depuis l'intérieur des canaux internes (55).

6. Système selon l'une quelconque des revendications 1 à 3, le mécanisme à cliquet comprenant des bosses ondulées (92) sur un segment venant en prise avec des bosses ondulées (94) appariées sur l'autre segment ; en particulier comprenant en outre un manchon de compression (96) entourant chacune des régions chevauchées sur des côtés opposés des segments antérieur et postérieur (42, 44 ; 82, 84).

7. Système selon l'une quelconque des revendications 1 à 6, comprenant en outre une valve hémostatique positionnée au niveau d'une extrémité proximale de la gaine d'accès (70), le système comprenant également une suture en bourse (136) conçue pour fermer hermétiquement une incision d'accès dans l'oreillette gauche autour de la gaine d'accès (70) ; et/ou chacun des dispositifs d'ajustement de tension (138a, 138b) présentant un cadran rotatif destiné à ajuster la tension sur les filaments (46a, 46b) d'ajustement associés et une jauge (140) étalonnée pour indiquer la distance sur laquelle le filament (46a, 46b) d'ajustement associé est déplacé.

8. Procédé de simulation d'une implantation d'un anneau d'annuloplastie (30) chez un patient simulé au niveau d'un anneau de valvule cardiaque native et d'ajustement d'une forme périphérique de l'anneau d'annuloplastie (30), comprenant :
l'arrêt du cœur d'un patient simulé et la réalisation d'une voie d'accès à un anneau de valvule cardiaque native à travers un corps vers le cœur, comprenant une incision d'accès dans le cœur conduisant à un anneau de valvule cardiaque ;
le bouclage d'une pluralité de sutures d'ancrage à travers l'anneau de valvule cardiaque à des emplacements écartés et la rétraction des extrémités libres des sutures d'ancrage hors du corps ;
le passage d'une extrémité de chacune des paires d'extrémités libres des sutures d'ancrage à travers une interface périphérique perméable à la suture d'un anneau d'annuloplastie (30), l'anneau d'annuloplastie (30) comprenant un noyau interne (40 ; 80) définissant une forme périphérique continue autour d'une ouverture centrale et l'interface entourant étroitement le noyau interne (40 ; 80) et présentant une forme appariée, le noyau interne (40 ; 80) présentant un segment antérieur (42 ; 82) arqué qui chevauche un segment postérieur (44 ; 84) arqué sur des côtés opposés, la forme périphérique étant une forme arrondie en D dans une vue en plan, à la fois le segment antérieur (42 ; 82) et le segment postérieur (44 ; 84) s'élevant à partir des côtés opposés afin de former une forme de selle et les segments antérieur et postérieur (42, 44 ; 82, 84) définissant un mécanisme à cliquet au niveau de régions chevauchées sur des côtés opposés de telle sorte qu'une position du segment antérieur (42, 82) par rapport au segment postérieur (44, 84) peut être modifiée et la forme périphérique de l'anneau d'annuloplastie (30) peut être ajustée, l'anneau d'annuloplastie (30) comprenant en outre une paire de filaments (46a, 46b) d'ajustement conçus pour passer à travers des passages dans un segment du noyau interne (40 ; 80), les filaments (46a, 46b) étant reliés à l'autre segment et déplaçant celui-ci sur des côtés opposés afin d'ajuster la forme périphérique de l'anneau d'annuloplastie (30) ;
le passage des deux filaments (46a, 46b) d'ajustement à travers une gaine d'accès (70), la gaine d'accès (70) et les filaments (46a, 46b) d'ajustement présentant des longueurs suffisantes pour s'étendre hors du corps, chacun des filaments (46a, 46b) d'ajustement passant séparément à travers un dispositif d'ajustement de tension (138a, 138b) afin de permettre un déplacement séparé des filaments (46a, 46b) d'ajustement par rapport à l'anneau d'annuloplastie (30) ;
l'avancement de l'anneau d'annuloplastie (30) vers l'anneau de valvule cardiaque à l'aide de la gaine d'accès (70) le long des sutures d'ancrage ;
la ligature des sutures d'ancrage sur un côté proximal de l'anneau d'annuloplastie (30) ;
la fermeture hermétique de l'incision d'accès autour de la gaine d'accès (70) ;
le redémarrage du cœur du patient ;
la visualisation externe du flux sanguin à travers l'anneau de valvule cardiaque ;
l'ajustement de la tension sur l'un des filaments (46a, 46b) d'ajustement ou les deux jusqu'à ce que la régurgitation à travers l'anneau de valvule cardiaque soit réduite au minimum ;
le sectionnement des filaments (46a, 46b) d'ajustement sur un côté proximal de l'anneau d'annuloplastie (30) ; et
le retrait des filaments (46a, 46b) d'ajustement et de la gaine d'accès (70) du corps et la fermeture de la voie d'accès.

9. Procédé selon la revendication 8, les passages étant formés dans le segment antérieur (42 ; 82) et ouverts vers deux trous d'entrée étroitement écartés sur un côté auriculaire d'une section médiane (50 ; 85) correspondante, les passages étant conçus de telle sorte que les filaments (46a, 46b) d'ajustement divergent à partir des trous d'entrée (60) et émergent sur un côté radialement vers l'extérieur de la section médiane (50 ; 85), des passages secondaires étant formés dans le segment antérieur (42 ; 82) de telle sorte que les filaments (46a, 46b) d'ajustement s'étendent dans des canaux internes (55) formés par des sections latérales (64 ; 86) du segment antérieur (42 ; 82), des extrémités libres du segment postérieur (44 ; 84) chevauchant le segment antérieur (42 ; 82) dans les canaux internes (55) et les filaments (46a, 46b) d'ajustement s'attachant aux extrémités libres du segment postérieur (44 ; 84) ; comprenant en outre en particulier un ressort de sollicitation positionné à l'intérieur de chacun des canaux internes (55) qui applique une force de compression à la fois au segment antérieur (42 ; 82) et au segment postérieur (44 ; 84) tendant à forcer leur écartement.

10. Procédé selon l'une quelconque des revendications 8 ou 9, le mécanisme à cliquet comprenant des dents sur un segment venant en prise avec un cran d'arrêt sur l'autre segment et chaque section latérale du segment antérieur comprenant le cran d'arrêt faisant saillie dans chacun des canaux internes (55) et le segment postérieur (44 ; 84) comprenant une section médiane et deux sections latérales se terminant au niveau des extrémités libres, chacune des sections latérales du segment postérieur (44 ; 84) présentant une série des dents avec lesquelles les crans d'arrêt viennent en prise dans chacun des canaux internes (55), les crans d'arrêt et les dents étant agencés pour empêcher la rétraction de la section latérale du segment postérieur (44 ; 84) sur chaque côté depuis l'intérieur des canaux internes (55).

11. Procédé selon l'une quelconque des revendications 8 ou 9, le mécanisme à cliquet comprenant des dents (66) sur un segment venant en prise avec un cran d'arrêt (58) sur l'autre segment.

12. Procédé selon l'une quelconque des revendications 8 ou 9, le mécanisme à cliquet comprenant des bosses ondulées (92) sur un segment venant en prise avec des bosses ondulées appariées sur l'autre segment ; en particulier comprenant en outre un manchon de compression entourant chacune des régions chevauchées sur des côtés opposés des segments antérieur et postérieur.

13. Procédé selon l'une quelconque des revendications 8 à 12, comprenant en outre une valve hémostatique positionnée au niveau d'une extrémité proximale de la gaine d'accès (70), le système comprenant également une suture en bourse (136) conçue pour fermer hermétiquement une incision d'accès dans l'oreillette gauche autour de la gaine d'accès (70).

14. Procédé selon l'une quelconque des revendications 8 à 13, chacun des dispositifs d'ajustement de tension (138a, 138b) présentant un cadran rotatif destiné à ajuster la tension sur les filaments (46a, 46b) d'ajustement associés et une jauge (140) étalonnée pour indiquer la distance sur laquelle le filament (46a, 46b) d'ajustement associé est déplacé.

15. Procédé selon l'une quelconque des revendications 8 à 14, comprenant en outre l'utilisation d'un poussoir de nœud (150) destiné à faire avancer des nœuds (156) uniques entre les filaments (46a, 46b) d'ajustement à travers la gaine d'accès (70) en vue de créer un nœud composite entre les filaments (46a, 46b) d'ajustement sur un côté proximal de l'anneau d'annuloplastie (30) avant le sectionnement des filaments (46a, 46b) d'ajustement.
